# EUROPEAN PATENT APPLICATION

(11) **EP 3 750 990 A1**
(43) Date of publication of application: **16.12.2020**
(21) Application number: 18213898.2
(22) Date of filing: 19.12.2018
(51) Int. Cl.: C12N 9/10, C12N 9/88, C12N 9/90

(54) **NOVEL POLYPEPTIDE-MODIFYING ENZYMES AND USES THEREOF**

(71) Applicant: ETH Zurich, 8092 Zurich (CH)
(72) Inventor: PIEL, Jörn, 8032 Zürich (CH); BHUSHAN, Agneya, 8005 Zürich (CH)

(57) **Abstract**

The present invention is directed to all aspects of novel polypeptide-modifying enzymes from an enzyme cluster in *Microvirgula aerodenitrificans.* The present invention also relates to nucleic acids encoding these enzymes as well as corresponding vectors and host cells comprising these. Moreover, the present invention encompasses the use of said enzymes in methods for modifying (poly)peptides of interest.

SEQUENCE LISTING

<110>  ETH Zurich

<120>  NOVEL POLYPEPTIDE-MODIFYING ENZYMES AND USES THEREOF

<130>  50707EP

<160>  21

<170>  PatentIn version 3.5

<210>  1
<211>  2085
<212>  DNA
<213>  Microvirgula aerodenitrificans

<400>  1
atgtgggaaa gcaacggatt gccggaagcg cctgatgact ccccggccct gcacgccggc      60

gagtcctgtt ccggcggcat cgacatggtg ttcgtgtgca tgccgtacgc cgccgtggaa     120

cgtccgtcgc tggccctcgg cacgctgact gcggtgcttg agcgcgaagg tctgtccagc     180

cgggcgatct acgccaatct cgaatttgcc gcccgcgtcg gccggcaggc ctatgaagtc     240

gtcaacaact ccgaaatcac gctccagctc ggcgaatgga cgttctccga agcggtattc     300

ggacagcagg gcgacatcga cgcctttatc aagggcctgg tcagctgtgg ctacaccgag     360

accggcctgc gggagctgct gcagggcctg cgcctcgagg ccgcccgcta tctcgacgag     420

ctggcgcagc gtgtcctggc cctgcagccg cgcattgtcg gctgcacgtc catgttccag     480

cagcactgcg cgtccctggg gctgcttcag cgcatccggg cgcagtcccc gggtacggtg     540

accatgctgg gcggcgcaaa ctgcgaaggg gaaatgggcg cggccaccca caggcaatat     600

ccatgggtcg actttgtcgt gtccggagaa gccgacaaat tgctgcccga actctgcaga     660

cgcattctgg cccggggcgc gggcattccg gtccatcacc tgcccgaggg cgtgctcggc     720

ccggcctcgc gccgcgtcct tgtcgttgcg ggcgccgcgg cagcaccggc ggtcggtcgg     780

gcttcgatta ccgatctcga cgaattgccg attccgaact tcgacgacta tttcgagcag     840

ctgcaagcct cgccgctgca cggctacgtg attcccggcc tgctgatcga aacctcccgc     900

ggttgctggt ggggcgccaa gcaccactgc acgttctgcg gcctgaacgg ttcgggcatg     960

gcgtttcgcg ccaagtcgca agcgcgagtc cagcaagaag tcagccagct ggcggcgcgc    1020

taccggctca agcggttcat ggcggtcgac aatattctcg acaacaaata cttctcccag    1080

gttctgccct ttctggccga agccggcgac atgctgtggt tctacgaaac caaggccaac    1140

ctcacccgca cccaggtcag cttgctgtcg caggccgggg tgcgctggat tcagccgggt    1200

atcgaggcga tggacgacgg cctgctgaag ctgctgcgca agggctgctc gaccgttatc    1260

aatgtgcagc tgctgaagtg ggcctacgac tacggcgtgt gggtgatgtg gaaccacctg    1320

cacggcgcgc cgggcgaaga tcccgagtgg tatgagcaca ttgccgactg gctgccgctg    1380

attgcgcacc tgcagccgcc atcgggcggc tccatgaccc gcatccgctt cgaccgtttc 1440

agcccctact tcaatgaaca ggccgacttc gccctggatc tcaagccctg ctggggctat 1500

ggccaggtct atcccgtgcc ggaaaagcag ctcgaacagc aggcctactt cttccgcaat 1560

gacggccact ccgccccgac gccgacccgg ctggcggcaa tgctgagcga gtgggccacc 1620

cgcttctacg cgccgactac cagggcaacc acgctgcccc ggcggagtga tgacgccccg 1680

gtgctggcct gggtcgccag cggctaccgg cagacggtcc gcgatacccg gccctgtgcc 1740

gtcgggtcgc tgcacgaact cagcgagctg gaaggacagg tctgtcatgc cctcgacagt 1800

gcgcagcatc tgcagggtct ggtgcaggcc ctgcgccatg ccggcagcac ggtcccggaa 1860

agggaaatcg gctccgcgct gcaacggctg gtcgatctga aaatcattgc cgaattcaac 1920

ggcaagttcc tttgcctggt caccagcgag aacccggtgc cctacaagtc gttctccgag 1980

tttgccggcg gcatgttcag ccttaccccc acacaccgga caccgccgaa gcccgaaacc 2040

ccatgggatg tttccttgag ggagttgttt gtctcttcca cgtaa 2085

<210> 2
<211> 694
<212> PRT
<213> Microvirgula aerodenitrificans

<400> 2

Met Trp Glu Ser Asn Gly Leu Pro Glu Ala Pro Asp Asp Ser Pro Ala
1               5                   10                  15

Leu His Ala Gly Glu Ser Cys Ser Gly Gly Ile Asp Met Val Phe Val
            20                  25                  30

Cys Met Pro Tyr Ala Ala Val Glu Arg Pro Ser Leu Ala Leu Gly Thr
        35                  40                  45

Leu Thr Ala Val Leu Glu Arg Glu Gly Leu Ser Ser Arg Ala Ile Tyr
    50                  55                  60

Ala Asn Leu Glu Phe Ala Ala Arg Val Gly Arg Gln Ala Tyr Glu Val
65                  70                  75                  80

Val Asn Asn Ser Glu Ile Thr Leu Gln Leu Gly Glu Trp Thr Phe Ser
                85                  90                  95

Glu Ala Val Phe Gly Gln Gln Gly Asp Ile Asp Ala Phe Ile Lys Gly
            100                 105                 110

Leu Val Ser Cys Gly Tyr Thr Glu Thr Gly Leu Arg Glu Leu Leu Gln
        115                 120                 125

Gly Leu Arg Leu Glu Ala Ala Arg Tyr Leu Asp Glu Leu Ala Gln Arg
    130                 135                 140

Val Leu Ala Leu Gln Pro Arg Ile Val Gly Cys Thr Ser Met Phe Gln
145                 150                 155                 160

Gln His Cys Ala Ser Leu Gly Leu Leu Gln Arg Ile Arg Ala Gln Ser
                165                 170                 175

Pro Gly Thr Val Thr Met Leu Gly Gly Ala Asn Cys Glu Gly Glu Met
            180                 185                 190

Gly Ala Ala Thr His Arg Gln Tyr Pro Trp Val Asp Phe Val Val Ser
        195                 200                 205

Gly Glu Ala Asp Lys Leu Leu Pro Glu Leu Cys Arg Arg Ile Leu Ala
    210                 215                 220

Arg Gly Ala Gly Ile Pro Val His His Leu Pro Glu Gly Val Leu Gly
225                 230                 235                 240

Pro Ala Ser Arg Arg Val Leu Val Val Ala Gly Ala Ala Ala Ala Pro
                245                 250                 255

Ala Val Gly Arg Ala Ser Ile Thr Asp Leu Asp Glu Leu Pro Ile Pro
            260                 265                 270

Asn Phe Asp Asp Tyr Phe Glu Gln Leu Gln Ala Ser Pro Leu His Gly
        275                 280                 285

Tyr Val Ile Pro Gly Leu Leu Ile Glu Thr Ser Arg Gly Cys Trp Trp
    290                 295                 300

Gly Ala Lys His His Cys Thr Phe Cys Gly Leu Asn Gly Ser Gly Met
305                 310                 315                 320

Ala Phe Arg Ala Lys Ser Gln Ala Arg Val Gln Gln Glu Val Ser Gln
                325                 330                 335

Leu Ala Ala Arg Tyr Arg Leu Lys Arg Phe Met Ala Val Asp Asn Ile
            340                 345                 350

Leu Asp Asn Lys Tyr Phe Ser Gln Val Leu Pro Phe Leu Ala Glu Ala
        355                 360                 365

Gly Asp Met Leu Trp Phe Tyr Glu Thr Lys Ala Asn Leu Thr Arg Thr

370                 375                 380

Gln Val Ser Leu Leu Ser Gln Ala Gly Val Arg Trp Ile Gln Pro Gly
385                 390                 395                 400

Ile Glu Ala Met Asp Asp Gly Leu Leu Lys Leu Leu Arg Lys Gly Cys
                405                 410                 415

Ser Thr Val Ile Asn Val Gln Leu Leu Lys Trp Ala Tyr Asp Tyr Gly
            420                 425                 430

Val Trp Val Met Trp Asn His Leu His Gly Ala Pro Gly Glu Asp Pro
        435                 440                 445

Glu Trp Tyr Glu His Ile Ala Asp Trp Leu Pro Leu Ile Ala His Leu
    450                 455                 460

Gln Pro Pro Ser Gly Gly Ser Met Thr Arg Ile Arg Phe Asp Arg Phe
465                 470                 475                 480

Ser Pro Tyr Phe Asn Glu Gln Ala Asp Phe Ala Leu Asp Leu Lys Pro
                485                 490                 495

Cys Trp Gly Tyr Gly Gln Val Tyr Pro Val Pro Glu Lys Gln Leu Glu
            500                 505                 510

Gln Gln Ala Tyr Phe Phe Arg Asn Asp Gly His Ser Ala Pro Thr Pro
        515                 520                 525

Thr Arg Leu Ala Ala Met Leu Ser Glu Trp Ala Thr Arg Phe Tyr Ala
    530                 535                 540

Pro Thr Thr Arg Ala Thr Thr Leu Pro Arg Arg Ser Asp Asp Ala Pro
545                 550                 555                 560

Val Leu Ala Trp Val Ala Ser Gly Tyr Arg Gln Thr Val Arg Asp Thr
                565                 570                 575

Arg Pro Cys Ala Val Gly Ser Leu His Glu Leu Ser Glu Leu Glu Gly
            580                 585                 590

Gln Val Cys His Ala Leu Asp Ser Ala Gln His Leu Gln Gly Leu Val
        595                 600                 605

Gln Ala Leu Arg His Ala Gly Ser Thr Val Pro Glu Arg Glu Ile Gly
    610                 615                 620

Ser Ala Leu Gln Arg Leu Val Asp Leu Lys Ile Ile Ala Glu Phe Asn
625                 630                 635                 640

Gly Lys Phe Leu Cys Leu Val Thr Ser Glu Asn Pro Val Pro Tyr Lys
                645                 650                 655

Ser Phe Ser Glu Phe Ala Gly Gly Met Phe Ser Leu Thr Pro Thr His
            660                 665                 670

Arg Thr Pro Pro Lys Pro Glu Thr Pro Trp Asp Val Ser Leu Arg Glu
        675                 680                 685

Leu Phe Val Ser Ser Thr
    690

<210>  3
<211>  1536
<212>  DNA
<213>  Microvirgula aerodenitrificans

<400>  3
atgcccacgc cacaagggac aagctacgcc gaaccgcaac accctgccgc cgacgccacg      60

ccggactggc cgcccatcac gatcgggcag gccaagcgcg ttctggaatg gtggtcttcg     120

tctgcggtgt tccgtgaact ggtggcgacc gatccggaac gcgccggccg cgactacaaa     180

ctgggcttca gtcccgaact gatccgtccg ctgtgggacg accgctatca cctcgacgcc     240

gccaacaagg atcgcccgca acacccgatc gttgccgagt accgggctta ttaccacacc     300

aagacgcagt ggcgcgacga ggtcaaaagg gagtgcgccc cggacgagcc ccgcctgaaa     360

acctggcgta cccgccagat cgcccgcaat gcgatggaaa acggtcttta cgacaacagc     420

atcattcact cgccgctggc catcgagctc agcgatggct gttcggtcgg ttgctggttc     480

tgcggcgtcg gcgcgaccag gtttgttgag acctgggact acaccgagga aaacgccacg      540

ctgtggcgcg gcgtgctgag cgtattgcac gacaagatcg gcgatgccag caaatggggg     600

ttctgctact gggccaccga cccgctggac aatccggact acgagcactt cgccagcgat     660

tttgccgata tcaccggcat gttcccgcag accaccaccg cccagggcca caaggacccg     720

gaacgggtgc tcaagctgct caggctgtcc gaatcgcgcg gctgcaaggt caaccgcttc     780

tcggtcctga ccgaatcgct gctgcgccgg attcacgatg catacacggc agatgagctg     840

acccaggtcg agattgtcgc ccagatgcgt gacgctaccg tgccgaaggc cgacgccggc     900

tcattccggg taaaggccag gaagactgcc aatgtcgtgg agcgggaaaa gaaaaaactg     960

attccgatcg ccgtgtccga gaacgggacg gaagacagcg acaagcccgc gctgaccatg     1020

cagcagccgg gcaccatcgc ctgcgttacc ggcttcctgc tcaatatggt ccggcgctcg    1080

gtcaagctga tcagtccctg ccgtgcttcc gagcaatggc cgctcggtta catcgtgttc    1140

gaggaatgca cgttcaccga cgccgccgac ctggaacgca agatcgaagc catgatcgag 1200

acccacatgc cgcaggagct cacgccggac gacccgatcc agctcaatcc gagcttccgc 1260

ctggagccgg tagccaacgg tttccgcgtg ctttccgacc tgagctgcat cgagttctcc 1320

cgccccaccc ctgacctggt cgagtacctc ggcgcgctgg gcgagcacgt gacatcaggc 1380

aagcgtacgg ccggtgaaat cgccgtgtcc tcgctgttct gctatggcgt gccggagaca 1440

aacaccctga gcaccctcgg cgcgatgctg aacctcggga tcctggtcga tgccaggggc 1500

cgcattaccg gccaatcagc agtggaagcc caatga 1536

<210> 4
<211> 511
<212> PRT
<213> Microvirgula aerodenitrificans

<400> 4

Met Pro Thr Pro Gln Gly Thr Ser Tyr Ala Glu Pro Gln His Pro Ala
1               5                   10                  15

Ala Asp Ala Thr Pro Asp Trp Pro Pro Ile Thr Ile Gly Gln Ala Lys
            20                  25                  30

Arg Val Leu Glu Trp Trp Ser Ser Ser Ala Val Phe Arg Glu Leu Val
        35                  40                  45

Ala Thr Asp Pro Glu Arg Ala Gly Arg Asp Tyr Lys Leu Gly Phe Ser
    50                  55                  60

Pro Glu Leu Ile Arg Pro Leu Trp Asp Asp Arg Tyr His Leu Asp Ala
65                  70                  75                  80

Ala Asn Lys Asp Arg Pro Gln His Pro Ile Val Ala Glu Tyr Arg Ala
                85                  90                  95

Tyr Tyr His Thr Lys Thr Gln Trp Arg Asp Glu Val Lys Arg Glu Cys
            100                 105                 110

Ala Pro Asp Glu Pro Arg Leu Lys Thr Trp Arg Thr Arg Gln Ile Ala
        115                 120                 125

Arg Asn Ala Met Glu Asn Gly Leu Tyr Asp Asn Ser Ile Ile His Ser
    130                 135                 140

Pro Leu Ala Ile Glu Leu Ser Asp Gly Cys Ser Val Gly Cys Trp Phe
145                 150                 155                 160

Cys Gly Val Gly Ala Thr Arg Phe Val Glu Thr Trp Asp Tyr Thr Glu
                165                 170                 175

Glu Asn Ala Thr Leu Trp Arg Gly Val Leu Ser Val Leu His Asp Lys
            180                 185                 190

Ile Gly Asp Ala Ser Lys Trp Gly Phe Cys Tyr Trp Ala Thr Asp Pro
        195                 200                 205

Leu Asp Asn Pro Asp Tyr Glu His Phe Ala Ser Asp Phe Ala Asp Ile
    210                 215                 220

Thr Gly Met Phe Pro Gln Thr Thr Thr Ala Gln Gly His Lys Asp Pro
225                 230                 235                 240

Glu Arg Val Leu Lys Leu Leu Arg Leu Ser Glu Ser Arg Gly Cys Lys
                245                 250                 255

Val Asn Arg Phe Ser Val Leu Thr Glu Ser Leu Leu Arg Arg Ile His
            260                 265                 270

Asp Ala Tyr Thr Ala Asp Glu Leu Thr Gln Val Glu Ile Val Ala Gln
        275                 280                 285

Met Arg Asp Ala Thr Val Pro Lys Ala Asp Ala Gly Ser Phe Arg Val
    290                 295                 300

Lys Ala Arg Lys Thr Ala Asn Val Val Glu Arg Glu Lys Lys Lys Leu
305                 310                 315                 320

Ile Pro Ile Ala Val Ser Glu Asn Gly Thr Glu Asp Ser Asp Lys Pro
                325                 330                 335

Ala Leu Thr Met Gln Gln Pro Gly Thr Ile Ala Cys Val Thr Gly Phe
            340                 345                 350

Leu Leu Asn Met Val Arg Arg Ser Val Lys Leu Ile Ser Pro Cys Arg
        355                 360                 365

Ala Ser Glu Gln Trp Pro Leu Gly Tyr Ile Val Phe Glu Glu Cys Thr
    370                 375                 380

Phe Thr Asp Ala Ala Asp Leu Glu Arg Lys Ile Glu Ala Met Ile Glu
385                 390                 395                 400

Thr His Met Pro Gln Glu Leu Thr Pro Asp Asp Pro Ile Gln Leu Asn
                405                 410                 415

Pro Ser Phe Arg Leu Glu Pro Val Ala Asn Gly Phe Arg Val Leu Ser
            420                 425                 430

Asp Leu Ser Cys Ile Glu Phe Ser Arg Pro Thr Pro Asp Leu Val Glu
        435                 440                 445

Tyr Leu Gly Ala Leu Gly Glu His Val Thr Ser Gly Lys Arg Thr Ala
    450                 455                 460

Gly Glu Ile Ala Val Ser Ser Leu Phe Cys Tyr Gly Val Pro Glu Thr
465                 470                 475                 480

Asn Thr Leu Ser Thr Leu Gly Ala Met Leu Asn Leu Gly Ile Leu Val
                485                 490                 495

Asp Ala Arg Gly Arg Ile Thr Gly Gln Ser Ala Val Glu Ala Gln
            500                 505                 510

<210>  5
<211>  1830
<212>  DNA
<213>  Microvirgula aerodenitrificans

<400>  5
atggccggtg cgcgggcgcc cttgctgcac gaactgttgc acgccgcacc ggcggaaacc      60

ggcgcgcgga tgagcgatac gctgtatcgc cgctggccgg cgctgctggg caccgacacg     120

ctgtccaggc ggctggactg gggctatgaa ggccccgatc cagccgatgc cccaccgtgg      180

gaagacatgc tggcggcggc gctgtccgca ccggacgacg ccgccctgcc cggcagtctc     240

gaccccatcg ggccgattcc gttcgaggaa gcgctgctgc ccttcgtcgc cgccgcacgc     300

accgggctgg agcgcgaagc gggtacggca ctcggcatct gctccccggc ggcgcgagcg     360

gcgctggaac gccatctgtt cgcgctgctc tccgtcgttg ccactccggt gctcggttcc     420

gcgttttcct tgcagcgcgc gctggccggc tcattgccct ggctgccccc ccggggtcgc     480

cagaactacg aagcctttgt cgcttcgctg caggccggtg gtctgcgcct gctgctggca     540

gactatcccg cactggcgcg cctgctggcg actgtggccc ggggatgggt gctggcccac     600

ggccggcttc tgcagcggct ggaaagggac tggcaccgga tcgtcgccac gttcggcttc     660

cccgccgccg actgtcagct ggacgggatc acggccggct gctccgaccc ccatgacggg     720

gggcagagcg taaccgttct gcacctgtcg aatggccggc gcctggtcta caagcccagg     780

tcgctggaga tggagaacgg gctggcgcaa ctgctcgact gggccggtcg caccggcttt     840

ccgtggccat ttcgcaccgt ggcgttgctg cccggcgagg gctatggctg gatggagttc     900

gtcgaggcgg cgccctgcga cgacgaggcg gccgtcgggc gtttccatgc ccgctcaggc     960

ggactgttct gcctgtggtg gctgctgcag ggaaccgacg tccaccacga aaacctgatc 1020

gccagcgggg agtatcccgt gatcgtcgat gccgagaccc tgctgcatcc ccgccccttt 1080

cccggcgtga ttcaccagtt gggcccgggc gccggttacg gtgcaagccc ggaggatgac 1140

tttgcccggg cactgctgga gagcggtttc ctggctaccg gcaaggcgct cgatctgtcg 1200

gcctggggac aggccgggga cggggccacg ccgtttcagg tcgccagctg ccaggccatc 1260

aattccgatg ccatgaccat ggcgcacgag acctttcatg tcgcaccgcg tcccaacctg 1320

ccggtgctgc acggccagcc ccggcaggcg gcggcgcatg ccggggcgat cgtcgacggg 1380

ttcacggcga tgtaccggct ggtcgtgcag caccgccatg actttctgac cctgcttgat 1440

ggcttcgccg gttgcagcgg gcgctttgtc gcgcgcgcga ccaataccta cgggctgctg 1500

ctgcatgccg gcctgcaacc ggaatggctg cgcgaaggcc ctgcccgcgg cgtgcttttc 1560

gagcgcctgc gccaggcggc gctggcggcc ccggcacgcc ctgcgtgctg gcccctgctc 1620

gacgccgaac tggcggcgct ggaacggctc gacatccccc gcctgtcctg ccgctgcgac 1680

ctgcccgcgc cctgctggcc ggcgccactg gatcaggccc gtgccagagt tgcgagtgcc 1740

tcgctgcctg atctcgaccg gcatgcggca gcactccggc aagcactgac accccagacc 1800

gcctctcccc gccaccctca cccagcctga 1830

<210> 6
<211> 609
<212> PRT
<213> Microvirgula aerodenitrificans

<400> 6

Met Ala Gly Ala Arg Ala Pro Leu Leu His Glu Leu Leu His Ala Ala
1               5                   10                  15

Pro Ala Glu Thr Gly Ala Arg Met Ser Asp Thr Leu Tyr Arg Arg Trp
            20                  25                  30

Pro Ala Leu Leu Gly Thr Asp Thr Leu Ser Arg Arg Leu Asp Trp Gly
        35                  40                  45

Tyr Glu Gly Pro Asp Pro Ala Asp Ala Pro Pro Trp Glu Asp Met Leu
    50                  55                  60

Ala Ala Ala Leu Ser Ala Pro Asp Asp Ala Ala Leu Pro Gly Ser Leu
65                  70                  75                  80

Asp Pro Ile Gly Pro Ile Pro Phe Glu Glu Ala Leu Leu Pro Phe Val
                85                  90                  95

Ala Ala Ala Arg Thr Gly Leu Glu Arg Glu Ala Gly Thr Ala Leu Gly

            100                 105                 110

Ile Cys Ser Pro Ala Ala Arg Ala Ala Leu Glu Arg His Leu Phe Ala
        115                 120                 125

Leu Leu Ser Val Val Ala Thr Pro Val Leu Gly Ser Ala Phe Ser Leu
    130                 135                 140

Gln Arg Ala Leu Ala Gly Ser Leu Pro Trp Leu Pro Pro Arg Gly Arg
145                 150                 155                 160

Gln Asn Tyr Glu Ala Phe Val Ala Ser Leu Gln Ala Gly Gly Leu Arg
                165                 170                 175

Leu Leu Leu Ala Asp Tyr Pro Ala Leu Ala Arg Leu Leu Ala Thr Val
            180                 185                 190

Ala Arg Gly Trp Val Leu Ala His Gly Arg Leu Leu Gln Arg Leu Glu
        195                 200                 205

Arg Asp Trp His Arg Ile Val Ala Thr Phe Gly Phe Pro Ala Ala Asp
    210                 215                 220

Cys Gln Leu Asp Gly Ile Thr Ala Gly Cys Ser Asp Pro His Asp Gly
225                 230                 235                 240

Gly Gln Ser Val Thr Val Leu His Leu Ser Asn Gly Arg Arg Leu Val
                245                 250                 255

Tyr Lys Pro Arg Ser Leu Glu Met Glu Asn Gly Leu Ala Gln Leu Leu
            260                 265                 270

Asp Trp Ala Gly Arg Thr Gly Phe Pro Trp Pro Phe Arg Thr Val Ala
        275                 280                 285

Leu Leu Pro Gly Glu Gly Tyr Gly Trp Met Glu Phe Val Glu Ala Ala
    290                 295                 300

Pro Cys Asp Asp Glu Ala Ala Val Gly Arg Phe His Ala Arg Ser Gly
305                 310                 315                 320

Gly Leu Phe Cys Leu Trp Trp Leu Leu Gln Gly Thr Asp Val His His
                325                 330                 335

Glu Asn Leu Ile Ala Ser Gly Glu Tyr Pro Val Ile Val Asp Ala Glu
            340                 345                 350

Thr Leu Leu His Pro Arg Pro Phe Pro Gly Val Ile His Gln Leu Gly
        355                 360                 365

Pro Gly Ala Gly Tyr Gly Ala Ser Pro Glu Asp Asp Phe Ala Arg Ala
    370                 375                 380

Leu Leu Glu Ser Gly Phe Leu Ala Thr Gly Lys Ala Leu Asp Leu Ser
385                 390                 395                 400

Ala Trp Gly Gln Ala Gly Asp Gly Ala Thr Pro Phe Gln Val Ala Ser
                405                 410                 415

Cys Gln Ala Ile Asn Ser Asp Ala Met Thr Met Ala His Glu Thr Phe
            420                 425                 430

His Val Ala Pro Arg Pro Asn Leu Pro Val Leu His Gly Gln Pro Arg
        435                 440                 445

Gln Ala Ala Ala His Ala Gly Ala Ile Val Asp Gly Phe Thr Ala Met
    450                 455                 460

Tyr Arg Leu Val Val Gln His Arg His Asp Phe Leu Thr Leu Leu Asp
465                 470                 475                 480

Gly Phe Ala Gly Cys Ser Gly Arg Phe Val Ala Arg Ala Thr Asn Thr
                485                 490                 495

Tyr Gly Leu Leu Leu His Ala Gly Leu Gln Pro Glu Trp Leu Arg Glu
            500                 505                 510

Gly Pro Ala Arg Gly Val Leu Phe Glu Arg Leu Arg Gln Ala Ala Leu
        515                 520                 525

Ala Ala Pro Ala Arg Pro Ala Cys Trp Pro Leu Leu Asp Ala Glu Leu
    530                 535                 540

Ala Ala Leu Glu Arg Leu Asp Ile Pro Arg Leu Ser Cys Arg Cys Asp
545                 550                 555                 560

Leu Pro Ala Pro Cys Trp Pro Ala Pro Leu Asp Gln Ala Arg Ala Arg
                565                 570                 575

Val Ala Ser Ala Ser Leu Pro Asp Leu Asp Arg His Ala Ala Ala Leu
            580                 585                 590

Arg Gln Ala Leu Thr Pro Gln Thr Ala Ser Pro Arg His Pro His Pro
        595                 600                 605

Ala

<210> 7
<211> 1155
<212> DNA
<213> Microvirgula aerodenitrificans

<400> 7
atgaccccgc cactcgccac caccattgac cggctccgcg actaccttga ccgggtcggc 60

ttccagcaga tctacaaata cattgtcgcg gtcaaccatt acgccgtgac gccggcgctg 120

atcacgcgca acactgccgc cagtgtccac cacttcttcg atagccggct gggcggcagg 180

gcggaattcg ccctgttgca gtgcctgatg accgggcgtc cggccgagca tgcagcgctg 240

ccggacaagg accgggcgct ggccgatgcg ctggtgacgg ccggcctgct gcgggccagt 300

ccggatgggc gggaagtgtc cggcgcggac cggcagctga tttcggcctt cggcgtggat 360

ctgctgatcg accgccgcat tcatttcggc ggcgaagtcc acgaggtgta tatcggtccc 420

gacagctact ggatgctgta ttacatcaat gcttccggta ttgcccgcac gcatcgcgcc 480

gtcgacctgt gcaccggcag cggcattgcc gcgctgtatc tgtcgctgtt taccgatcat 540

gttctggcga ccgatatcgg cgatgtgccg ctggcgctgg tcgagataaa ccgccgcctg 600

aaccggcgcg acgccggcac gatggagatc cggcgcgaga acctgaacga cacgctggat 660

ggccgtgaac gcttcgacct gctcacctgc aacccgcctt tcgtggcctt ccctcccggc 720

tacagcggca cgctctattc gcagggcacc ggcgtcgacg gactcggcta catgcgcgac 780

atcgtcggcc gcctgccgga agtgctcaat cccggcggtt ctgcctacct cgtggcggat 840

ctctgcggcg atgcgcacgg cccgcacttc ctgggtgagc tggagagcat ggtcaccggg 900

cacggcatgc gcatcgaggc gttcatcgac catgtcctgc cggcctcggc ccaggtcggc 960

ccgatctcgg acttcctgag acacgcagcc gggctgcctg cggacaccga catcgcggca 1020

gacgtgcagg cgttccagcg cgagacgctg cgcgccgact actactacct gacgacgatc 1080

cgcctgcaaa cggccgcgca gaaccccgga ctgcgcatgc tgcgacgcga cccgctcccc 1140

ggggccggga cgtga 1155

<210> 8
<211> 384
<212> PRT
<213> Microvirgula aerodenitrificans

<400> 8

Met Thr Pro Pro Leu Ala Thr Thr Ile Asp Arg Leu Arg Asp Tyr Leu
1               5                   10                  15

Asp Arg Val Gly Phe Gln Gln Ile Tyr Lys Tyr Ile Val Ala Val Asn
            20                  25                  30

His Tyr Ala Val Thr Pro Ala Leu Ile Thr Arg Asn Thr Ala Ala Ser
        35                  40                  45

Val His His Phe Phe Asp Ser Arg Leu Gly Gly Arg Ala Glu Phe Ala
    50                  55                  60

Leu Leu Gln Cys Leu Met Thr Gly Arg Pro Ala Glu His Ala Ala Leu
65                  70                  75                  80

Pro Asp Lys Asp Arg Ala Leu Ala Asp Ala Leu Val Thr Ala Gly Leu
                85                  90                  95

Leu Arg Ala Ser Pro Asp Gly Arg Glu Val Ser Gly Ala Asp Arg Gln
            100                 105                 110

Leu Ile Ser Ala Phe Gly Val Asp Leu Leu Ile Asp Arg Arg Ile His
        115                 120                 125

Phe Gly Gly Glu Val His Glu Val Tyr Ile Gly Pro Asp Ser Tyr Trp
    130                 135                 140

Met Leu Tyr Tyr Ile Asn Ala Ser Gly Ile Ala Arg Thr His Arg Ala
145                 150                 155                 160

Val Asp Leu Cys Thr Gly Ser Gly Ile Ala Ala Leu Tyr Leu Ser Leu
                165                 170                 175

Phe Thr Asp His Val Leu Ala Thr Asp Ile Gly Asp Val Pro Leu Ala
            180                 185                 190

Leu Val Glu Ile Asn Arg Arg Leu Asn Arg Arg Asp Ala Gly Thr Met
        195                 200                 205

Glu Ile Arg Arg Glu Asn Leu Asn Asp Thr Leu Asp Gly Arg Glu Arg
    210                 215                 220

Phe Asp Leu Leu Thr Cys Asn Pro Pro Phe Val Ala Phe Pro Pro Gly
225                 230                 235                 240

Tyr Ser Gly Thr Leu Tyr Ser Gln Gly Thr Gly Val Asp Gly Leu Gly
                245                 250                 255

Tyr Met Arg Asp Ile Val Gly Arg Leu Pro Glu Val Leu Asn Pro Gly
            260                 265                 270

Gly Ser Ala Tyr Leu Val Ala Asp Leu Cys Gly Asp Ala His Gly Pro
        275                 280                 285

His Phe Leu Gly Glu Leu Glu Ser Met Val Thr Gly His Gly Met Arg
    290                 295                 300

Ile Glu Ala Phe Ile Asp His Val Leu Pro Ala Ser Ala Gln Val Gly
305                 310                 315                 320

Pro Ile Ser Asp Phe Leu Arg His Ala Ala Gly Leu Pro Ala Asp Thr
                325                 330                 335

Asp Ile Ala Ala Asp Val Gln Ala Phe Gln Arg Glu Thr Leu Arg Ala
            340                 345                 350

Asp Tyr Tyr Tyr Leu Thr Thr Ile Arg Leu Gln Thr Ala Ala Gln Asn
        355                 360                 365

Pro Gly Leu Arg Met Leu Arg Arg Asp Pro Leu Pro Gly Ala Gly Thr
    370                 375                 380

<210>  9
<211>  300
<212>  DNA
<213>  Microvirgula aerodenitrificans

<400>  9
atgactacga ctacaccggc cagcacccag gttccgcaaa cccggcgcga tctggaaacc      60

cacatcatca ccaaggcctg gaaggatccc gagtacaagg cccagctgct caaggacccg     120

aaggcggcgc tgcaggatgc gctcaagagc attgacccgt ccctctccct gcccgactcg     180

ctgcaggtcc aggtgcacga ggagaacgcc aacctgttcc accttgtgct gccgcgcaat     240

ccgagcgaga tctcgctcgc cgaggtggta ggcgacaacc ttgaagccgt ggcaccgcaa     300

<210>  10
<211>  100
<212>  PRT
<213>  Microvirgula aerodenitrificans

<400>  10

Met Thr Thr Thr Thr Pro Ala Ser Thr Gln Val Pro Gln Thr Arg Arg
1               5                   10                  15

Asp Leu Glu Thr His Ile Ile Thr Lys Ala Trp Lys Asp Pro Glu Tyr
            20                  25                  30

Lys Ala Gln Leu Leu Lys Asp Pro Lys Ala Ala Leu Gln Asp Ala Leu
        35                  40                  45

Lys Ser Ile Asp Pro Ser Leu Ser Leu Pro Asp Ser Leu Gln Val Gln
    50                  55                  60

Val His Glu Glu Asn Ala Asn Leu Phe His Leu Val Leu Pro Arg Asn
65                  70                  75                  80

Pro Ser Glu Ile Ser Leu Ala Glu Val Val Gly Asp Asn Leu Glu Ala
                85                  90                  95

Val Ala Pro Gln
            100

<210>  11
<211>  54
<212>  PRT
<213>  Candidatus Entotheonella factor

<400>  11

Gln Ala Ala Gly Gly Thr Gly Ile Gly Val Val Val Ala Val Val Ala
1               5                   10                  15

Gly Ala Val Ala Asn Thr Gly Ala Gly Val Asn Gln Val Ala Gly Gly
            20                  25                  30

Asn Ile Asn Val Val Gly Asn Ile Asn Val Asn Ala Asn Val Ser Val
        35                  40                  45

Asn Met Asn Gln Thr Thr
    50

<210>  12
<211>  51
<212>  PRT
<213>  Microvirgula aerodenitrificans

<400>  12

Ala Val Ala Pro Gln Thr Ile Ala Val Val Leu Val Ala Val Val Gly
1               5                   10                  15

Ala Ala Ala Ala Ala Val Val Thr Tyr Leu Gly Ala Ala Asn Val Val
            20                  25                  30

Gly Ala Ala Asn Gly Thr Val Thr Ala Asn Ala Val Ala Asn Thr Asn
        35                  40                  45

Ala Val Ala
    50

<210>  13
<211>  55
<212>  PRT
<213>  Rhodospirillaceae bacterium BRH_c57

<400>  13

Ala Val Ala Pro Gln Thr Ile Ala Val Val Val Ala Val Val Gly Ile
1               5                   10                  15

Gly Val Val Ala Gly Asn Thr Leu Gly Val Val Asn Asn Val Gly Ala
            20                  25                  30

Gly Asn Ala Val Ala Ala Gly Asn Val Ala Thr Thr Gly Asn Ala Val
        35                  40                  45

Ala Asn Thr Asn Val Ile Ala
    50                  55

<210>  14
<211>  53
<212>  PRT
<213>  Rhodospirillaceae bacterium BRH_c57

<400>  14

Ala Val Ala Pro Gln Thr Ile Ala Val Val Val Ala Ala Leu Gly Val
1               5                   10                  15

Val Val Ala Asn Thr Leu Gly Ala Val Asn Asn Val Gly Ala Gly Asn
            20                  25                  30

Ala Val Thr Val Gly Asn Val Ala Thr Thr Gly Asn Ala Val Ala Asn
        35                  40                  45

Ser Thr Ser Val Ser
    50

<210>  15
<211>  38
<212>  PRT
<213>  Rhodospirillaceae bacterium BRH_c57

<400>  15

Ala Val Ala Pro Gln Thr Ile Ala Val Val Thr Asn Gly Val Gly Val
1               5                   10                  15

Cys Ala Val Val Thr Gly Pro Val Thr Ile Ala Tyr Pro Thr Asn Val
            20                  25                  30

Val Thr Cys Val Val Ala

        35

<210>  16
<211>  52
<212>  PRT
<213>  Verrucomicrobia bacterium SCGC AAA164-I21

<400>  16

Ala Val Ala Gly Gly Val Ala Ala Ile Ala Val Phe Val Val Gly Val
1               5                   10                  15

Val Ala Val Ala Val Gly Gly Thr Val Thr Val Ala Val Asn Ile Asn
            20                  25                  30

Ala Ala Val Asn Val His Thr Val Val Asn Ala Val Lys Gly Ala Asn
        35                  40                  45

Glu Ser Pro Trp
    50

<210>  17
<211>  46
<212>  PRT
<213>  Microvirgula aerodenitrificans

<400>  17

Thr Ile Ala Val Val Leu Val Ala Val Val Gly Ala Ala Ala Ala Ala
1               5                   10                  15

Val Val Thr Tyr Leu Gly Ala Ala Asn Val Val Gly Ala Ala Asn Gly
            20                  25                  30

Thr Val Thr Ala Asn Ala Val Ala Asn Thr Asn Ala Val Ala
        35                  40                  45

<210>  18
<211>  51
<212>  PRT
<213>  Microvirgula aerodenitrificans

<400>  18

Ala Val Ala Gly Gly Thr Ile Ala Val Val Leu Val Ala Val Val Gly
1               5                   10                  15

Ala Ala Ala Ala Ala Val Val Thr Tyr Leu Gly Ala Ala Asn Val Val
            20                  25                  30

Gly Ala Ala Asn Gly Thr Val Thr Ala Asn Ala Val Ala Asn Thr Asn
        35                  40                  45

Ala Val Ala
    50

<210>  19
<211>  55
<212>  PRT
<213>  Microvirgula aerodenitrificans

<400>  19

Ala Val Ala Pro Gln Thr Ile Ala Val Val Val Ala Val Val Gly Ile
1               5                   10                  15

Gly Val Val Ala Gly Asn Thr Leu Gly Val Val Asn Asn Val Gly Ala
            20                  25                  30

Gly Asn Ala Val Ala Ala Gly Asn Val Ala Thr Thr Gly Asn Ala Val
        35                  40                  45

Ala Asn Thr Asn Val Ile Ala
    50                  55

<210>  20
<211>  53
<212>  PRT
<213>  Microvirgula aerodenitrificans

<400>  20

Ala Val Ala Pro Gln Thr Ile Ala Val Val Val Ala Ala Leu Gly Val
1               5                   10                  15

Val Val Ala Asn Thr Leu Gly Ala Val Asn Asn Val Gly Ala Gly Asn
            20                  25                  30

Ala Val Thr Val Gly Asn Val Ala Thr Thr Gly Asn Ala Val Ala Asn
        35                  40                  45

Ser Thr Ser Val Ser
    50

<210>  21
<211>  54
<212>  PRT
<213>  Microvirgula aerodenitrificans

<400>  21

Ala Val Ala Pro Gln Thr Gly Ile Gly Val Val Val Ala Val Val Ala
1               5                   10                  15

Gly Ala Val Ala Asn Thr Gly Ala Gly Val Asn Gln Val Ala Gly Gly

20                  25                  30

Asn Ile Asn Val Val Gly Asn Ile Asn Val Asn Ala Asn Val Ser Val
        35                  40                  45

Asn Met Asn Gln Thr Thr
    50

## Description

The present invention is directed to all aspects of novel polypeptide-modifying enzymes from an enzyme cluster in *Microvirgula aerodenitrificans.* The present invention also relates to nucleic acids encoding these enzymes as well as corresponding vectors and host cells comprising these. Moreover, the present invention encompasses the use of said enzymes in methods for modifying (poly)peptides of interest.

### Background of the invention

Many physiologically active polypeptide-based compounds in nature, for example sponge-related cytotoxins, feature post-translational modifications that have a strong impact on activity. In this respect, marine sponges are a treasure trove of bioactive natural products that exhibit a wide range of activities relevant for biomedical applications. But further development is often impeded by limited supply and synthetically challenging chemical structures. Biological strategies have been proposed for sustainable and economic production based on the suspected or known role of symbiotic bacteria as actual sources of many sponge compounds. However, to date these have not been implemented, mainly because the known producers remain uncultured, are only distantly related to established bacterial hosts for heterologous gene expression, and commonly use unconventional, poorly studied enzymes for natural product biosynthesis.

Among the most complex and biosynthetically unusual natural products known are the polytheonamides from the sponge *Theonella swinhoei.* These remarkable 49-residue peptides form a β-helical structure and insert into membranes as unimolecular pores, resulting in potent cytotoxicity at lower picomolar range. The chemical basis of this mechanism is the presence of numerous non-proteinogenic residues with lipophilic or other modifications, as well as an almost perfect alternation of d- and I-configured amino acids that is only interrupted by achiral Gly residues.

The polytheonamides with their unusual peptide structure are of ribosomal biosynthetic origin and belong to a new family of ribosomally synthesized and post-translationally modified peptides (RiPPs), termed proteusins.

It was found that when acting on PoyA, a precursor protein comprised of standard I-amino acids, only seven enzymes introduce a total of 50 posttranslational modifications in a highly promiscuous but precisely controlled fashion (Freeman et al. Nat. Chem. 9, 387-395, 2017). Like most other RiPPs (Arnison et al., Nat Prod Rep 30, 108-60, 2016), PoyA is organized into an N-terminal leader region and a C-terminal core that is post-translationally modified and ultimately released from the leader. As the earliest-acting modifying enzyme, one radical S-adenosylmethionine (rSAM) enzyme, PoyD, generates 18 D-amino acids by epimerization of the PoyA core. Further iterative enzymes install 8 N-methylations of Asn side chains (PoyE), 4 hydroxylations (Poyl), 1 dehydration at Thr (PoyF), and 17 methylations at diverse non-activated carbon atoms (PoyB and PoyC), including 4 methylations that together create a *t*-butyl unit (PoyC). Ultimately, proteolytic cleavage by PoyH releases the core and triggers hydrolysis of an N-terminal enamine function at the *t*-butylated Thr to generate the pharmacologically important α-keto moiety of polytheonamides.

Considerable challenges were encountered when attempting to reconstitute the complete enzymatic pathway in heterologous bacterial hosts.

For example, although the epimerase PoyD acts irreversibly at each amino acid center, its coproduction with PoyA in the bacterial host *E. coli* resulted in mixtures of peptide products that were only processed at the C-terminal half (Freeman et al., Nat. Chem. 9, 387-395, 2017). The most recalcitrant enzymes were the C-methyltransferases PoyB and PoyC, which remained completely inactive in *E. coli.* Both are cobalamin-dependent rSAM methyltransferases, a highly challenging protein family in the context of biotechnological applications (Lanz et al., Biochemistry 57, 1475-1490, 2018). Functional expressions of *poyB* and *poyC* were ultimately successful in the non-standard host *Rhizobi-um leguminosarum* (Freeman et al., Nat. Chem. 9, 387-395, 2017), which unlike *E. coli* contains a complete cobalamin biosynthetic pathway (Burton et al., Canadian Journal of Botany 30, 521-524, 1952). In this way, C-methylations occurred at most of the core positions, but with low efficiency and resulting in complex mixtures of mono- to tetra-methylated products.

Due to the challenges of identifying and expressing genes from invertebrate symbionts, biological synthesis has to date only been achieved for a single example, patellamide-type RiPPs from tuni-cate-associated cyanobacteria (Donia et al., Nat Chem Biol 2, 729-35, 2006).

Cobalamin (vitamin B12)-dependent radical S-adenosyl methionine (Cbl-dependent rSAM) enzymes catalyze some of the synthetically most challenging reactions, such as methylations of unactivated carbon centers. These proteins comprise a large superfamily of currently about 7000 known members (Bridwell-Rabb et al. Nature 544, 322-326 2017). Among the numerous examples of bacteria-derived bioactive natural products generated by such reactions (Huo et al. Chem Biol 19, 1278-1287, 2012) are the economically important carbapenems, thiostrepton, gentamicin, fosfomycin-type compounds, moenomycin, which are all commercial antibiotics. Heterologous efforts to produce such compounds either for overproduction or biosynthetic studies have, however, been limited to organisms of related strains. Some examples of these are fosfomycin (Woodyer et al. Chem Biol 13(11): 1171-1182, 2006), bottromycins and thiostreptons (Huo et al. Chem Biol 19, 1278-1287, 2012, Li et al. Mol. BioSyst. 2011,7, 82-90), all produced by *Streptomyces* species, where responsible clusters were transfered into a standard *Streptomyces* strain to produce these compounds. The Cbl-dependent rSAM methyltransferases involved in these cases, however, catalyze only up to two methylations.

It is the objective of the present invention to provide new enzymatic tools for the post-translational modification of polypeptides of interest, optionally for use in heterologous hosts, in particular in bacterial hosts, e.g. such as *E. coli.* Preferably, these enzyme tools catalyze at least one, optionally multiple C-methylations, N-methylations, epimerizations and/or dehydration(s) and optionally lead to homogenous product mixtures. These enzyme tools may have utility for preparing post-translationally modified physiologically active polypeptides, e.g. polypeptide antibiotics, polypeptide cytotoxins, polytheonamides, etc.

In a first aspect, the objective technical problem is solved by an isolated and purified nucleic acid, comprising or consisting of a nucleic acid sequence selected from the group consisting of:
(i) a nucleic acid sequence listed in any one of SEQ ID NOs: 1 (aerC), 3 (aerD), and 5 (aerF), 7 (aerE);
(ii) a nucleic acid sequence of at least 80 % or 90 % sequence identity, optionally at least 95 % or 98 % sequence identity with a nucleic acid sequence of (i), optionally over the whole sequence;
(iii) a nucleic acid sequence that hybridizes to the nucleic acid sequence of (i) or (ii) under stringent conditions;
(iv) a fragment of any of the nucleic acid sequences of (i) to (iii), that hybridizes to the nucleic acid sequence of (i) or (ii) under stringent conditions;
(v) a nucleic acid sequence degenerated with respect to the nucleic acid sequence of any of (i) to (iv);
(vi) a nucleic acid sequence, wherein said nucleic acid sequence is derivable by substitution, addition and/or deletion of at least one nucleic acid of the nucleic acid sequences of (i) to (v) that hybridizes to a nucleic acid sequence of (i) or (ii) under stringent conditions;
(vii) a nucleic acid sequence complementary to the nucleic acid sequence of any of (i) to (vi); wherein the nucleic acid sequence of any of (i) to (vii),
   (a) when based on SEQ ID NO: 1 (aerC) encodes a polypeptide that has cobalamin-dependent rSAM methyltransferase activity, optionally methylates one or more valine(s) to tert-leucine(s), methylates one or more isoleucine(s), methylates one or more leucine(s) and/or methylates one or more threonine(s);
   (b) when based on SEQ ID NO: 3 (aerD) encodes a polypeptide that has rSAM epimerase activity to convert one or more L-amino acid(s) into D-amino acid(s);
   (c) when based on SEQ ID NO: 5 (aerF) encodes a polypeptide that has dehydratase activity to dehydrate an N-terminal threonine and serine to an alpha-keto functional group; or
   (d) when based on SEQ ID NO: 7 (aerE) encodes a polypeptide that has asparagine (ASN) N-methyltransferase activity for methylating one or more side chain amines of one or more asparagine(s).

It was surprisingly found that the above nucleic acids derived from *Microvirgula denitrificans* encode fully functional enzymes that modify polypeptides of interest in a stable, efficient, and often in a repetitive manner, i.e. multiple modifications of the same polypeptide substrate, and which enzymes produce homogenous products. And even more surprisingly, the enzymes can function in heterologous organisms such as bacteria, e.g. *E. coli.*

The term "% (percent) sequence identity" as known to the skilled artisan and used herein in the context of nucleic acids indicates the degree of relatedness among two or more nucleic acid molecules that is determined by agreement among the sequences. The percentage of "sequence identity" is the result of the percentage of identical regions in two or more sequences while taking into consideration the gaps and other sequence peculiarities.

The identity of related nucleic acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two nucleic acid sequences comprise, but are not limited to, BLASTN (Altschul et al., (1990) J. Mol. Biol., 215:403-410) and LALIGN (Huang and Miller, (1991) Adv. Appl. Math., 12:337-357). The BLAST programs can be obtained from the National Center for Biotechnology Information (NCBI) and from other sources (BLAST handbook, Altschul et al., NCB NLM NIH Bethesda, MD 20894).

The nucleic acid molecules according to the invention may be prepared synthetically by methods well-known to the skilled person, but also may be isolated from suitable DNA libraries and other publicly available sources of nucleic acids and subsequently may optionally be mutated. The preparation of such libraries or mutations is well-known to the person skilled in the art.

The nucleic acid of the present invention may be a DNA, RNA or PNA, optionally DNA or PNA.

In some instances, the present invention also provides novel nucleic acids encoding the polypeptide enzymes of the present invention characterized in that they have the ability to hybridize to a specifically referenced nucleic acid sequence, optionally under stringent conditions. Next to common and/or standard protocols in the prior art for determining the ability to hybridize to a specifically referenced nucleic acid sequence under stringent conditions (e.g. Sambrook and Russell, (2001) Molecular cloning: A laboratory manual (3 volumes)), it is preferred to analyze and determine the ability to hybridize to a specifically referenced nucleic acid sequence under stringent conditions by comparing the nucleotide sequences, which may be found in gene databases (e.g. http://www.ncbi.nlm.nih.gov/entrez/query.fcgi?db=nucleotide and http://genome.jgi.doe.gov/programs/fungi/index.jsf) with alignment tools, such as e.g. the above-mentioned BLASTN (Altschul et al., (1990) J. Mol. Biol., 215:403-410), LALIGN alignment tools and multiple alignment tools such as e.g. CLUSTALW (Sievers F et al., (2011) Mol. Sys. Bio. 7: 539), MUSCLE (Edgar., (2004) Nucl. Acids Res. 32:1792-7) or T-COFFEE (Notredame et al., (2000) J of Mol. Bio 302 1: 205-17).

Most preferably, the ability of a nucleic acid of the present invention to hybridize to a specifically referenced nucleic acid, e.g. those listed in any of SEQ ID NOs 1, 3, 5 and 7, is confirmed in a Southern blot assay under the following conditions: 6x sodium chloride/sodium citrate (SSC) at 45°C followed by a wash in 0.2x SSC, 0.1% SDS at 65°C.

The term "nucleic acid encoding a polypeptide" as used in the context of the present invention is meant to include allelic variations and redundancies in the genetic code. For example, the term "a nucleic acid sequence degenerated with respect to the nucleic acid code" in the context of a specific nucleic acid sequence, e.g. SEQ ID NOs: 1, 3, 5 or 7, is meant to describe nucleic acids that differ from the specified sequence but encode the identical amino acid sequence.

The nucleic acids of the present invention code for specific polypeptide enzymes, in particular,
(a) a polypeptide that has cobalamin-dependent rSAM methyltransferase activity, optionally methylates one or more valine(s) to tert-leucine(s), methylates one or more isoleucine(s), methylates one or more leucine(s) and/or methylates one or more threonine(s);
(b) a polypeptide that has rSAM epimerase activity to convert one or more L-amino acid(s) into D-amino acid(s);
(c) a polypeptide that has dehydratase activity to dehydrate an N-terminal threonine or serine to an alpha-keto functional group; or
(d) a polypeptide that has asparagine (ASN) N-methyltransferase activity for methylating one or more side chain amines of one or more asparagine(s).

Therefore, in a further aspect, the invention relates to an isolated and purified polypeptide selected from the group consisting of:
(i) polypeptides comprising or consisting of an amino acid sequence selected from the group consisting of SEQ ID NOs: 2, 4, 6 and 8,
(ii) polypeptides encoded by any of the nucleic acids of claim 1;
(iii) polypeptides having an amino acid sequence identity of at least 70 % or 80 %; optionally at least 90 % or 95 % with the polypeptides of (i) and/or (ii); and
(iv) a functional fragment and/or functional derivative of (i), (ii) or (iii);
   wherein the polypeptide of any of (i) to (iv),
   (a) when based on an amino acid sequence of SEQ ID NO: 2 (AerC) has cobalamin-dependent rSAM methyltransferase activity, optionally methylates one or more valine(s) to tert-leucine(s), methylates one or more isoleucine(s), methylates one or more leucine(s) and/or methylates one or more threonine(s);
   (b) when based on an amino acid sequence of SEQ ID NO: 4 (AerD) has rSAM epimerase activity to convert one or more L-amino acid(s) into D-amino acid(s);
   (c) when based on an amino acid sequence of SEQ ID NO: 6 (AerF) has dehydratase activity to dehydrate an N-terminal threonine or serine to an alpha-keto functional group; or
   (d) when based on an amino acid sequence of SEQ ID NO: 8 (AerE) has asparagine (ASN) N-methyltransferase activity for methylating one or more side chain amine(s) of asparagine(s).
      The term "when based on" in conjunction with a specified amino acid sequence indicates that the polypeptide is one of the polypeptides defined in any of passages (i) to (iv) above.

The term (poly)peptide, as used herein, is meant to encompass peptides, polypeptides, oligopeptides and proteins that comprise two or more amino acids linked covalently through peptide bonds. The term does not refer to a specific length of the product. Optionally, the term (poly)peptide includes (poly)peptides with post-translational modifications, for example, glycosylations, acetylations, phosphorylations and the like, as well as (poly)peptides comprising non-natural or non-conventional amino acids and functional derivatives as described below. The term non-natural or non-conventional amino acid refers to naturally occurring or naturally not occurring unnatural amino acids or chemical amino acid analogues, e.g. D-amino acids, α,α-disubstituted amino acids, N-alkyl amino acids, homo-amino acids, dehydroamino acids, aromatic amino acids (other than phenylalanine, tyrosine and tryptophan), and ortho-, meta- or para-aminobenzoic acid. Non-conventional amino acids also include compounds which have an amine and carboxyl functional group separated in a 1,3 or larger substitution pattern, such as β-alanine, γ-amino butyric acid, Freidinger lactam, the bicyclic dipeptide (BTD), amino-methyl benzoic acid and others well known in the art. Statine-like isosteres, hydroxyethylene isosteres, reduced amide bond isosteres, thioamide isosteres, urea isosteres, carbamate isosteres, thioether isosteres, vinyl isosteres and other amide bond isosteres known to the art may also be used.

The percentage identity of related amino acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two amino acid sequences comprise, but are not limited to, TBLASTN, BLASTP, BLASTX, TBLASTX (Altschul et al., J. Mol. Biol., 215, 403-410, 1990), or ClustalW (Larkin MA et al., Bioinformatics, 23, 2947-2948, 2007). The BLAST programs can be obtained from the National Center for Biotechnology Information (NCBI) and from other sources (BLAST handbook, Altschul et al., NCB NLM NIH Bethesda, MD 20894). The ClustalW program can be obtained from http://www.clustal.org.

The term "functional derivative" of a (poly)peptide of the present invention is meant to include any (poly)peptide or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative still has at least some enzymatic activity to a measurable extent, e.g. of at least about 1 to 10%, preferably 10 to 50% enzymatic activity of the original unmodified (poly)peptide of the invention.

In this context a "functional fragment" of the invention is one that forms part of a (poly)-peptide or derivative of the invention and still has at least some enzymatic activity to a measurable extent, e.g. of at least about 1 to 10%, preferably 10 to 50% enzymatic activity of the original unmodified (poly)peptide of the invention.

The enzymatic polypeptides of the present invention can be used to modify substrate polypeptides with broad amino acid sequence variation. The enzymes can be isolated or partially purified before use. Specific antibodies can be used to identify, isolate, purify, localise or bind the enzymes of the present invention.

Therefore, in a further aspect the present invention also reads on an antibody, optionally a monoclonal antibody, a functional fragment or functional derivative thereof, or antibody-like binding protein that specifically binds a polypeptide of the invention.

Antibodies, functional fragments and functional derivatives thereof for practicing the invention are routinely available by hybridoma technology (Kohler and Milstein, Nature 256, 495-497, 1975), antibody phage display (Winter et al., Annu. Rev. Immunol. 12, 433-455, 1994), ribosome display (Schaffitzel et al., J. Immunol. Methods, 231, 119-135, 1999) and iterative colony filter screening (Giovannoni et al., Nucleic Acids Res. 29, E27, 2001) once the target antigen is available. Typical proteases for fragmenting antibodies into functional products are well-known. Other fragmentation techniques can be used as well as long as the resulting fragment has a specific high affinity and, preferably a dissociation constant in the micromolar to picomolar range.

A very convenient antibody fragment for targeting applications is the single-chain Fv fragment, in which a variable heavy and a variable light domain are joined together by a polypeptide linker. Other antibody fragments for vascular targeting applications include Fab fragments, Fab2 fragments, miniantibodies (also called small immune proteins), tandem scFv-scFv fusions as well as scFv fusions with suitable domains (e.g. with the Fc portion of an immuneglobulin). For a review on certain antibody formats, see Holliger P, Hudson PJ.; Engineered antibody fragments and the rise of single domains. Nat Biotechnol. 2005 Sep., 23(9):1126-36.).

The term "functional derivative" of an antibody for use in the present invention is meant to include any antibody or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative has substantially the same binding affinity as to its original antigen and, preferably, has a dissociation constant in the micro-, nano- or picomolar range. A most preferred derivative of the antibodies for use in the present invention is an antibody fusion protein that will be defined in more detail below.

In a preferred embodiment, the antibody, fragment or functional derivative thereof for use in the invention is one that is selected from the group consisting of polyclonal antibodies, monoclonal antibodies, chimeric antibodies, humanized antibodies, CDR-grafted antibodies, Fv-fragments, Fab-fragments and Fab2-fragments and antibody-like binding proteins, e.g. affilines, anticalines and aptamers.

For a review of antibody-like binding proteins see Binz et al. on engineering binding proteins from non-immunoglobulin domains in Nature Biotechnology, Vol. 23, No. 10, October 2005, 12571268. The term "aptamer" describes nucleic acids that bind to a polypeptide with high affinity. Aptamers can be isolated from a large pool of different single-stranded RNA molecules by selection methods such as SELEX (see, e.g., Jayasena, Clin. Chem., 45, p. 1628 - 1650, (1999); Klug and Famulok, M. Mol. Biol. Rep., 20, p. 97 - 107 (1994); US 5,582,981). Aptamers can also be synthesized and selected in their mirror form, for example, as the L-ribonucleotide (Nolte et al., Nat. Biotechnol., 14, pp.1116 - 1119, (1996); Klussmann et al., Nat. Biotechnol., 14, p. 1112 - 1115, (1996)). Forms isolated in this way have the advantage that they are not degraded by naturally occurring ribonucleases and, therefore, have a greater stability.

Another antibody-like binding protein and alternative to classical antibodies are the so-called "protein scaffolds", for example, anticalines, that are based on lipocaline (Beste et al., Proc. Natl. Acad. Sci. USA, 96, p. 1898 - 1903, (1999)). The natural ligand binding sites of lipocalines, for example, of the retinol-binding protein or bilin-binding protein, can be changed, for example, by employing a "combinatorial protein design" approach, and in such a way that they bind selected haptens (Skerra, Biochem. Biophys. Acta, 1482, pp. 337 - 350, (2000)). For other protein scaffolds it is also known that they are alternatives for antibodies (Skerra, J. Mol. Recognit, 13, pp. 167 - 287, (2000)). (Hey, Trends in Biotechnology, 23, pp. 514-522, (2005)).

According to the invention the term functional antibody derivative is meant to include said protein-derived alternatives for antibodies, i.e. antibody-like binding proteins, e.g. affilines, anticalines and aptamers that specifically recognize at least one extracellular domain of oncofetal fibronectin or oncofetal tenascin.

In summary, the terms antibody, functional fragment and functional derivative thereof denote all substances that have the same or similar specific binding affinity to any one of the extracellular domains of oncofetal fibronectin or oncofetal tenascin as a complete antibody having specific binding affinity to these targets.

The polypeptide enzymes of the present invention may be encoded and expressed by a vector, optionally a bacterial plasmid, comprising a nucleic acid of the present invention and optionally nucleic acids further encoding and expressing a polypeptide of interest for posttranslational modification by at least one enzymatic polypeptide of the present invention.

For example, vectors suitable for practicing the present invention may be selected from the group of vectors consisting of pLMB509, pLMB51, pK18mobSacB, pET 28b, pACYC DUET, pCDF DUET, pET DUET, pRSF DUET and pBAD vectors.

Unlike other sponge-related post-translationally modifying enzymes the enzymes of the present invention can be transferred functionally into bacterial host cells and stably and efficiently produce homogeneously modified polypeptides of interest.

In this regard, the present invention also provides for a bacterial host cell, optionally a bacterial host cell producing cobalamin, optionally *Microvirgula aerodenitricans* or *E. coli* host cell, optionally a cobalamin-producing *E. coli,* comprising at least one or more of the nucleic acids of the present invention, wherein the host cell expresses and modifies a heterologous polypeptide of interest by one or more polypeptides of the present invention. For example, the host cell for practicing the present invention may be selected from the group consisting of *Microvirgula* sp. AG722, *Microvirgula aerodenitrificans* strain BE2.4, *Microvirgula curvata, Microvirgula* sp. DB2-7, *Microvirgula* sp. H8, *Microvirgula* sp. HW7, *Cystobacter fucus*, *Rhizobium leguminosarum and Sinorhizobium meliloti.*

Even though the enzymes of the present invention exist naturally in *Microvirgula aerodenitrificans,* it was so far only speculated that this organism may actually express enzymes, for example, with cobalamin-dependent rSAM methyltransferase activity. And this assumption was based on the finding of sequence analogies only. Based on sequence analogy, these rSAM proteins comprise a large superfamily of currently about 7000 known members (Bridwell-Rabb et al. Nature 544, 322-326 2017). However, their activity, transferability into heterologous organisms, their substrate specificity and their ability to produce homogenous products differs widely.

In a further embodiment, the present invention relates to a *Microvirgula aerodenitrificans* host cell, wherein the host cell expresses at least one heterologous polypeptide for enzymatic modification and one or more polypeptides of the present invention, thereby modifying the at least one heterologous polypeptide by the one or more polypeptides. The *Microvirgula aerodenitrificans* host cell of the present invention clearly differs from the naturally occurring *Microvirgula aerodenitrificans* by the heterologous substrate polypeptide. This difference can be easily verified by sequence comparison of the nucleic acid sequences of a naturally occurring *Microvirgula aerodenitrificans* with the corresponding sequences of a recombinantly modified *Microvirgula aerodenitrificans* host cell. Alternatively, and when antibodies for the heterologous protein are available, both host cells can by lysed and the antibodies can be applied to specifically bind and identify the heterologous polypeptide.

For example, a *Microvirgula aerodenitrificans* host cell of the present invention may express
(i) at least one polypeptide based on amino acid sequence SEQ ID NO: 2 (AerC);
(ii) at least one polypeptide based on amino acid sequence SEQ ID NO: 4 (AerD);
(iii) at least one polypeptide based on amino acid sequence SEQ ID NO: 6 (AerF); and/or
(iv) at least one polypeptide based on amino acid sequence SEQ ID NO: 8 (AerE),
   with the *proviso* that expression of polypeptide (iv) requires the expression of polypeptide (ii).

It was found that the enzymatic activity of an asparagine N-methyltransferase based on SEQ ID NO: 8 requires the co-existence of an rSAM epimerase based on SEQ ID NO: 4. In another embodiment the host cell of the invention expresses at least the polypeptide of (iv) based on SEQ ID NO: 8 and at least the polypeptide of (ii) based on SEQ ID NO: 4).

In a further embodiment, the bacterial host cell of the present invention is an *Escherichia coli* host cell expressing
(i) at least one polypeptide based on amino acid sequence SEQ ID NO: 2 (AerC);
(ii) at least one polypeptide based on amino acid sequence SEQ ID NO: 4 (AerD);
(iii) at least one polypeptide based on amino acid sequence SEQ ID NO: 6 (AerF)2; and/or
(iv) at least one polypeptide based on amino acid sequence SEQ ID NO: 8 (AerE),
   with the proviso that (a) expression of polypeptide (iv) requires the expression of polypeptide (ii) and (b) expression of (i) requires bacterial production or supplement of cobalamin (preferably D and E).

The host cells of the present invention are particularly useful for preparing polypeptide-based antibiotics from polypeptide precursor substrates that can be produced recombinantly. For example, host cells of the present invention may be a *Microvirgula aerodenitrificans* or *Escherichia coli* host cell expressing a heterologous polypeptide for enzymatic modification selected from the group of polypeptide precursors of boceprevir, telapevir, glecaprevir, atazanavir, vancomycin, colistin, teixobactin, bacitracin, gramicidin A-D, goserelin, leuprolide, nateglidine, octreotide, thiostreptons, bottromycins, polymyxin, actinomycin, nisin, protegrin, dalbavancin, daptomycin, enfurvirtide, oritavancin, teicoplanin, and guavanin 2.

In wild type *Microvirgula aerodenitrificans* the natural substrate forms part of the AerA cluster together with a leader sequence featuring nucleic acid sequence SEQ ID NO: 9 and corresponding amino acid sequence SEQ ID NO: 10, which directs the substrate to the cytosol.

The invention also encompasses a *Microvirgula aerodenitrificans*, optionally an *Escherichia coli* host cell of the invention expressing a heterologous polypeptide for enzymatic modification encoded by a nucleic acid sequence comprised in the aerA cluster and encompassing the nucleic acid sequence of Seq. ID. NO.: 9 or a nucleic acid sequence hybridizing thereto under stringent conditions.

The present invention is also directed to a composition comprising at least one nucleic acid, at least one polypeptide, at least one vector, or at least one bacterial host cell of the present invention as described herein.

The nucleic acid sequences, amino acid sequences, vectors and host cells of the present invention have utility for use in a method for producing and modifying a heterologous polypeptide in a *Microvirgula aerodenitricans* cell or an *E. coli* cell, optionally a cobalamin-producing *E. coli* cell. For example, such method may comprise the steps of
(i) providing a *Microvirgula aerodenitricans* or *E. coli* host cell of the invention, optionally a cobalamin-producing *E. coli* host cell, functionally expressing
   a. at least one polypeptide enzyme of the invention and
   b. at least one heterologous polypeptide of interest; and
(ii) co-expressing the at least one polypeptide enzyme of the invention and the at least one heterologous polypeptide of interest;
(iii) and optionally co-expressing one or more further enzymes for modifying the at least one heterologous polypeptide of interest;
(iv) and optionally at least partially purifying the so-modified heterologous polypeptide, wherein the at least one polypeptide enzyme of the invention is capable of catalyzing at least one modification in the heterologous polypeptide.

Optionally, the method of the invention may comprise the steps of
(i) providing a *Microvirgula aerodenitricans* or a cobalamin-producing *E. coli* host cell, functionnally expressing
   a. at least one Cbl-dependent rSAM polypeptide enzyme of the invention and
   b. at least one heterologous polypeptide of interest; and
(ii) co-expressing the at least one Cbl-dependent rSAM enzyme and the at least one heterologous polypeptide;
(iii) and optionally co-expressing one or more further enzymes for modifying the heterologous polypeptide of interest;
(iv) and optionally at least partially purifying the so-modified heterologous (poly)peptide of interest;
wherein the at least one Cbl-dependent rSAM enzyme methylates one or more valine(s) to tert-leucine(s), methylates one or more isoleucine(s), methylates one or more leucine(s) and/or methylates one or more threonine(s) in the at least one heterologous polypeptide of interest.

For the above methods it is optional that the one or more further enzymes for modifying the heterologous polypeptide(s) in step (iii) are selected from the polypeptides of the invention.

In a further embodiment of the invention the method is one, wherein the one or more further enzymes for modifying the heterologous polypeptide(s) in step (iii) are selected from the group consisting of PoyB, PoyC (rSAM C-methyltransferases, Freeman et al., Nat. Chem. 9, 387-395, 2017), OspD, AvpD, PIpD, PoyD (Epimerases, Morinaka et. al. Angewandte Chemie, 56(3): 762-766, 2017), PIpXY (β-amino acid incorporation, Morinaka et. al. Science, 359, 779, 2018,), PtsY (S-methyltransferase, Helf et. al., ChemBioChem 18:444-450, 2017).

The method of the present invention is specifically suited for preparing polypeptide antibiotics from polypeptide precursors thereof.

For example, the methods of the invention can be used for modifying at least one heterologous polypeptide selected from the group consisting of polypeptide precursors of boceprevir, telapevir, glecaprevir, atazanavir, vancomycin, colistin, teixobactin, bacitracin, gramicidin A-D, goserelin, leuprolide, nateglidine, octreotide, thiostreptons, bottromycins polymyxin, actinomycin, nisin, protegrin, dalbavancin, daptomycin, enfurvirtide, oritavancin, teicoplanin and guavanin 2.

Another example for practicing the present invention is a method, wherein at least one, two or all of (i) the heterologous polypeptide(s) of interest, the polypeptide enzyme(s) of the present invention and/or the one or more further enzymes for modifying the heterologous polypeptide(s) are present in the form of host-integrated DNA and/or in the form of a plasmid.

The invention also relates to the products that are available for the first time with the enzymes, vectors, host cells and methods of the present invention.

For example, the invention encompasses a polypeptide, optionally a cytotoxin, an antibiotic polypeptide or antiviral polypeptide comprising a posttranslational modification selected from the group consisting of
(i) a methylation of one or more valine(s) to tert-leucine(s), a methylation of one or more isoleucine(s), a methylation of one or more leucine(s), a methylation of one or more threonine(s);
(ii) a conversion of one or more L-amino acid(s) into D-amino acid(s);
(iii) a hydrolyzation of an N-terminal dehydro-threonine or -serine to an alpha-keto functional group; and
(iv) a methylation of one or more side chain amine(s) of asparagine(s),
   wherein the polypeptide is obtained by a method of any of claims 12 to 17.

In this regard, the invention also pertains to the use of a nucleic acid, a polypeptide, an antibody, a vector, a host cell, composition or a method of the invention as described herein for modifying a heterologous polypeptide in a bacterial host cell or bacterially derived cell-free system.

The following Figures and Examples serve to illustrate the invention and are not intended to limit the scope of the invention as described in the appended claims.

### Figures

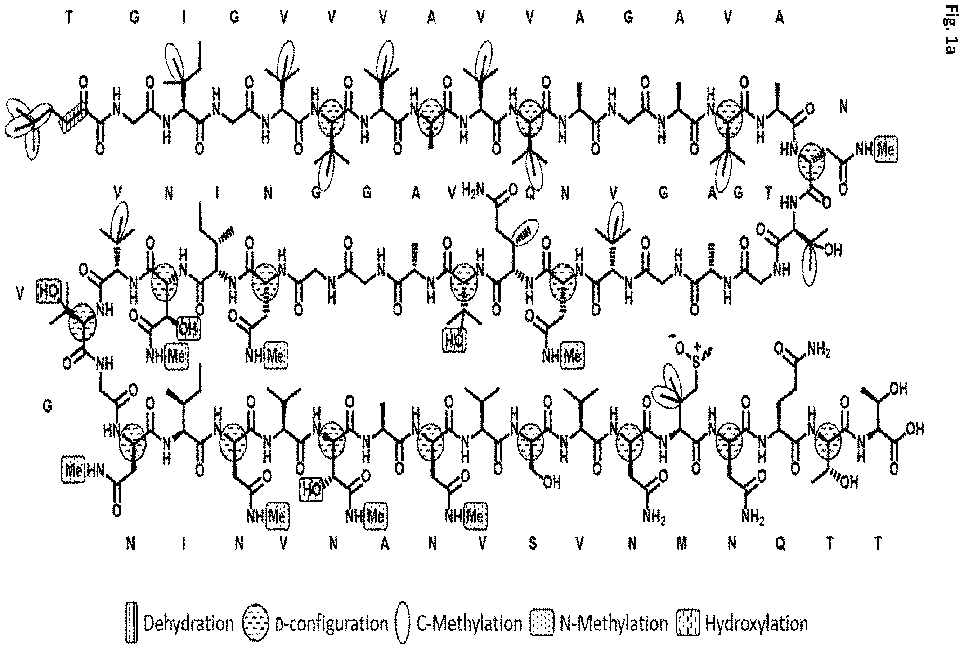

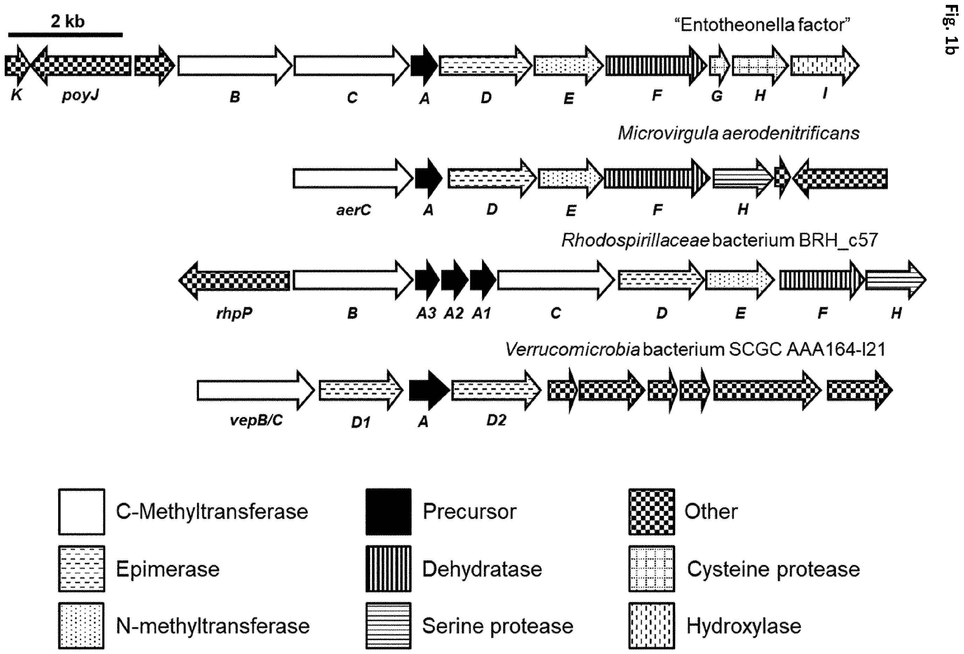

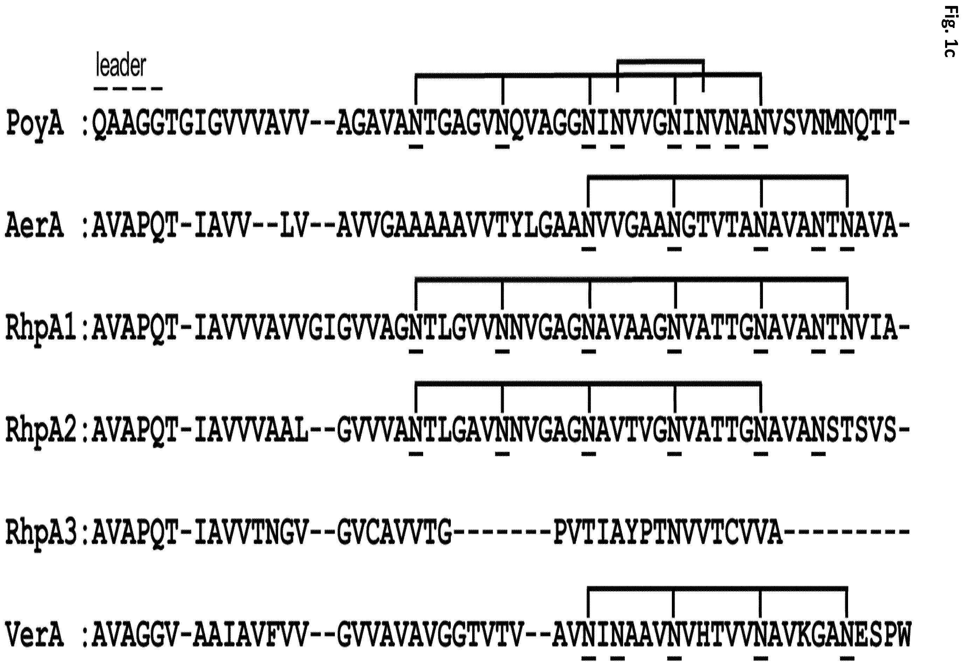

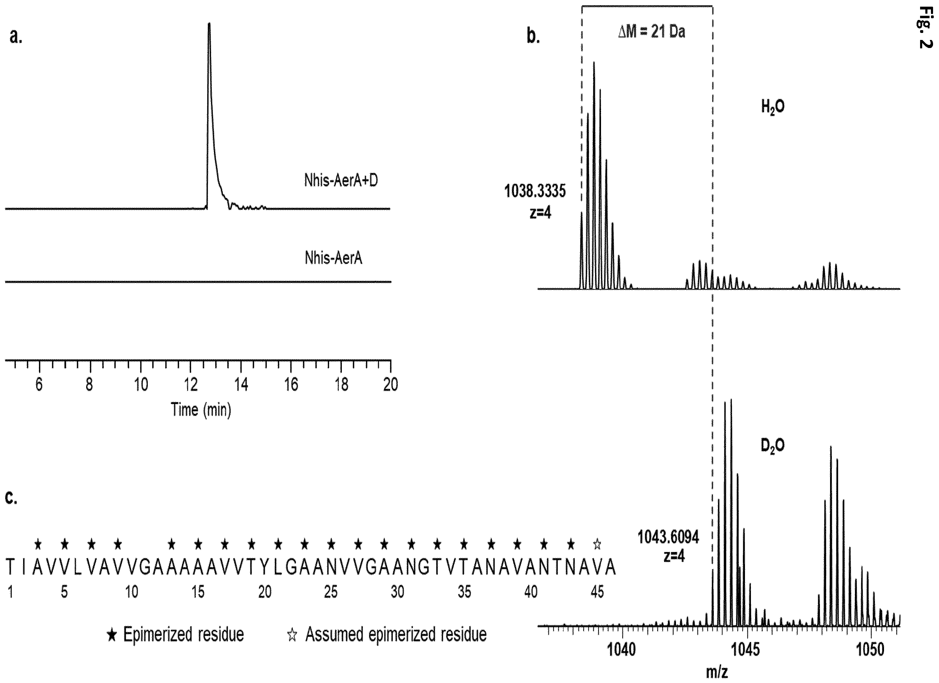

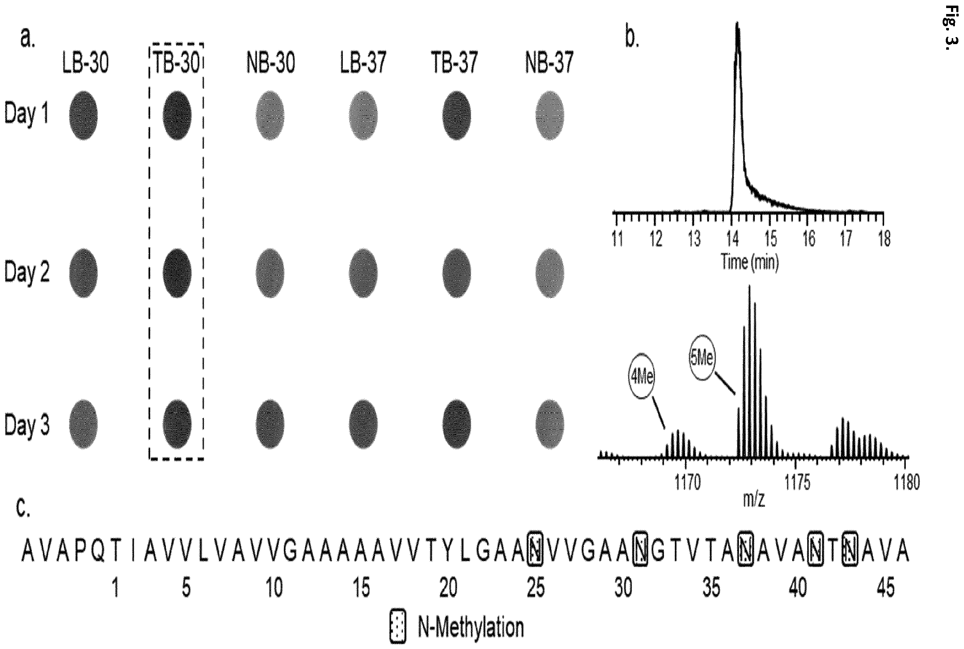

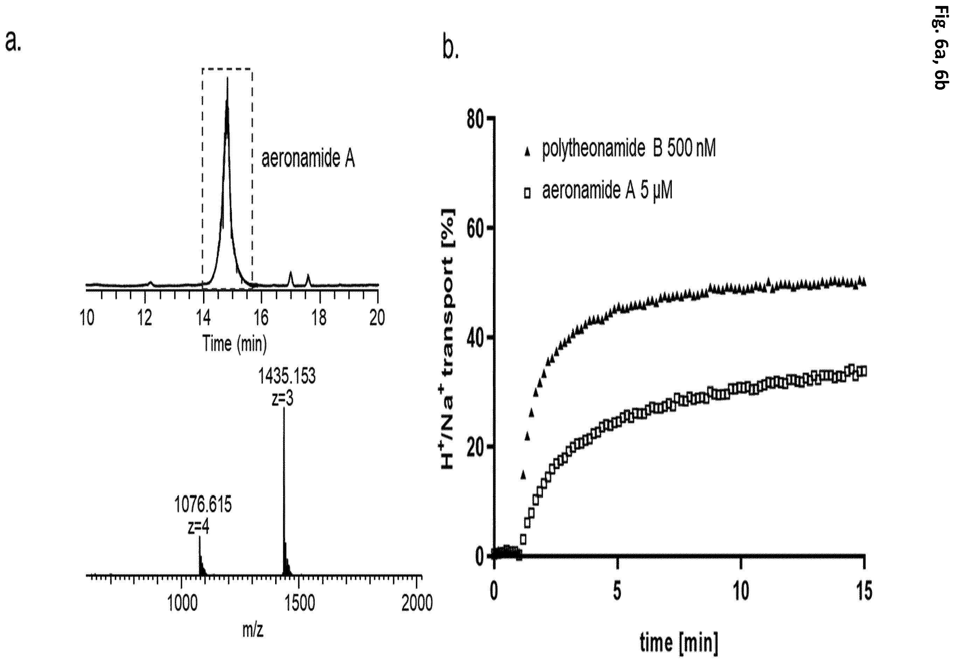

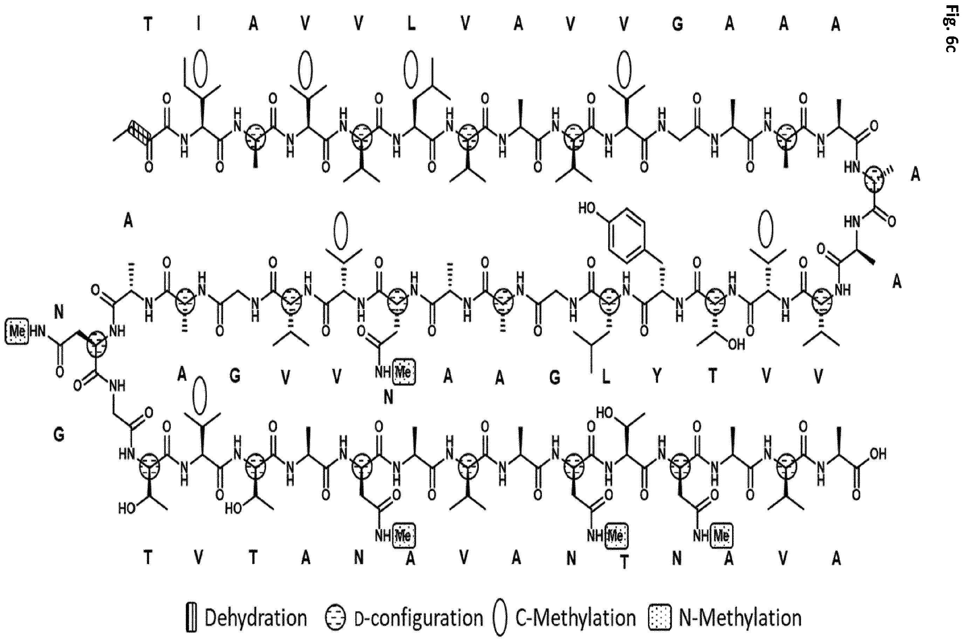

**Fig. 1** shows the structure and biosynthetic gene cluster of polytheonamides. (a) The secondary structure of polytheonamide A and B is shown, differing in the configuration of the methionine sulfoxide. Modifications occurring post-translationally on the canonical proteingenic amino acids are shown in the legend below. (b) Graphical representation of the polytheonamide (*poy*) gene cluster and comparison with similar candidate clusters (*aer*, *rhp* and *vep*) in other bacteria. Highlighting corresponds to the modifications hypothesized to occur from the encoded ORFs. (c) Alignment of the core sequences from all clusters. Asn residues predicted to be methylated are underlined, with predicted helix clamps shown.
   **PoyaA a.a. (SEQ ID NO: 11), *Candidatus Entotheonella factor***
      QAAGGTGIGVVVAVVAGAVANTGAGVNQVAGGNINVVGNINVNANVSVNMNQTT
   **AerA a.a.(SEQ ID NO: 12), *Microvirgula aerodenitrificans***
      AVAPQTIAVVLVAVVGAAAAAVVTYLGAANVVGAANGTVTANAVANTNAVA
   **RhoA1 a.a. (SEQ ID NO: 13), *Rhodospirillaceae* bacterium BRH_c57**
      AVAPQTIAVVVAVVGIGVVAGNTLGVVNNVGAGNAVAAGNVATTGNAVANTNVIA
   **RhoA2 a.a. (SEQ ID NO: 14), *Rhodospirillaceae* bacterium BRH_c57**
      AVAPQTIAVVVAALGVVVANTLGAVNNVGAGNAVTVGNVATTGNAVANSTSVS
   **RhoA3 a.a. (SEQ ID NO: 15), *Rhodospirillaceae* bacterium BRH_c57**
      AVAPQTIAVVTNGVGVCAVVTGPVTIAYPTNVVTCVVA
   **VerA a.a. (SEQ ID NO: 16), *Verrucomicrobia* bacterium SCGC AAA164-I21**
      AVAGGVAAIAVFVVGVVAVAVGGTVTVAVNINAAVNVHTVVNAVKGANESPW
**Fig 2****.** shows the (a) extracted ion chromatogram (EIC) looking for the protected AerA core following proteinase K digestion of Nhis-AerA and Nhis AerAD purified from *E. coli*; (b) ion of protected core fragment (top) after expressions in H₂O compared with ion observed following the same treatment after ODIS expressions (below). A mass shift of 21 Da was observed that was localized to the residues indicated (c). Assumed modifications refer to modifications that could not be localized to a particular residue during MS² analysis, but for which fragmentation support-ted the existence of such a modification.
   **Nhis-AerA a.a. (SEQ ID NO: 17) *Microvirgula aerodenitrificans***
   TIAVVLVAVVGAAAAAVVTYLGAANVVGAANGTVTANAVANTNAVA
**Fig 3****.** Shows the conditions for *aer* expression: (a) Results of the GusA assay in *M. aerodenitrificans.* Deeper blue color corresponds to stronger activity of the promoter, with TB-30 (dotted box) the best condition observed. LB- luria bertani medium, TB- terrific broth, NB- nutriaent broth. 30 and 37 correspond to temperature at which growth occurred. (b) EIC following cell-free assays showing product peak for GluC treated Nhis-AerA (5 methylations) and the corresponding mass spectra. (c) Position of methylations to Asn residues of the core based on MS-MS data. Methylation on Asn43 was not localized but proposed based on y-ion fragment masses observed. (SEQ ID NO: 12).

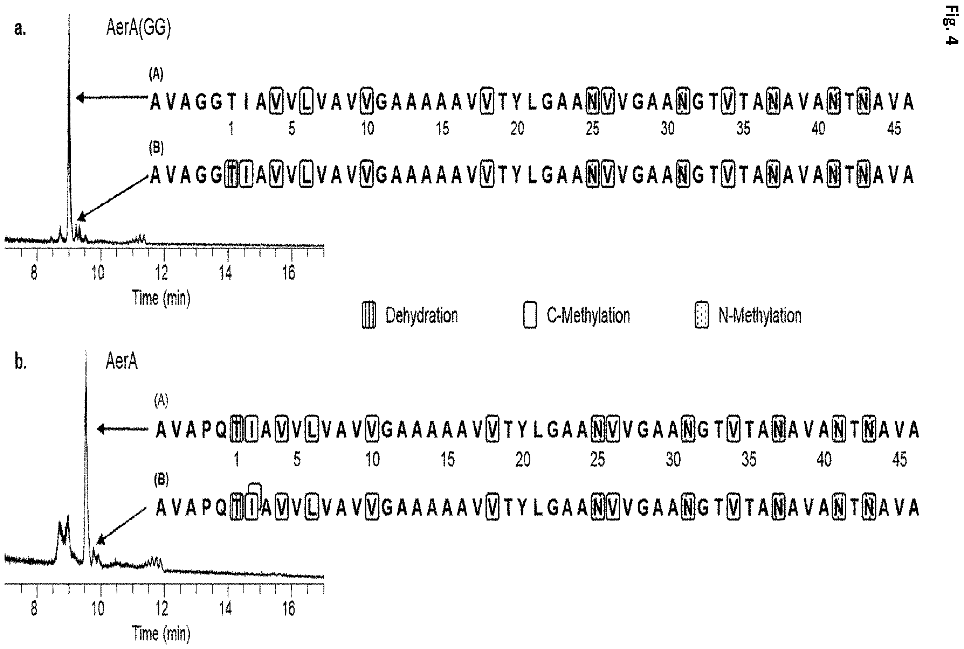

**Fig 4****.** shows the aeronamide characterization from expression in *M*. *aerodenitrificans.*
   (a) Total ion chromatogram (TIC) of GluC treated Nhis-AerA(GG). A: major product, B: minor product.
      **AerA(GG) a.a. (SEQ ID NO: 18) *Microvirgula aerodenitrificans***
      AVAGGTIAVVLVAVVGAAAAAVVTYLGAANVVGAANGTVTANAVANTNAVA
   (b) TIC of GluC treated Nhis-AerA. A: major product, B: minor product. Modifications localized to residues as described in the legend.
      **SEQ ID NO: 12, see above** **Fig 1C**

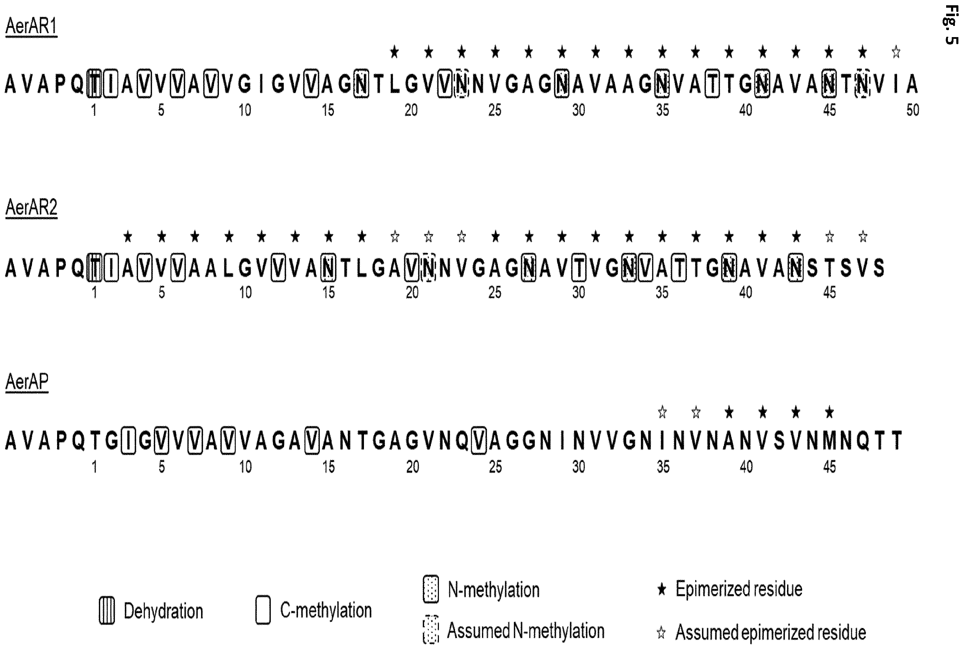

**Fig 5****.** Modifications localized to other cores expressed in *M. aerodenitrificans* using the tagged-bait strategy. Epimerizations localized via ODIS expressions in *E. coli.* Assumed modifications refer to modifications that could not be localized to a particular residue during MS² analysis, but for which fragmentation supported the existence of such a modification.
   **AerAR1 a.a. (SEQ ID NO: 19) *Microvirgula aerodenitrificans***
      AVAPQTIAVVVAVVGIGVVAGNTLGVVNNVGAGNAVAAGNVATTGNAVANTNVIA
   **AerAR2 a.a. (SEQ ID NO: 20) *Microvirgula aerodenitrificans***
      AVAPQTIAVVVAALGVVVANTLGAVNNVGAGNAVTVGNVATTGNAVANSTSVS
   **AerAP a.a. (SEQ ID NO: 21) *Microvirgula aerodenitrificans***
      AVAPQTGIGVVVAVVAGAVANTGAGVNQVAGGNINVVGNINVNANVSVNMNQTT
**Fig 6****.** (a) TIC (left) and mass spectra (right) of HPLC purified aeronamide A following *in vitro* cleavage of Nhis-AerA (from *M*. *aerodenitrificans*) with Nhis-AerH (from *E. coli*). (b) Results of H⁺/Na⁺ ion exchange activity assay on artificial liposomes for aeronamide A and polytheonamide B. (c) Structure of aeronamide A. Modified residues indicated according to the legend below. The orange balloons above the residues point to the residue a methylation was localized to, but without knowledge of the specific position of modification on the side chains.

### Examples

### Materials

Restriction enzymes, Q5 site-directed mutagenesis kit, and Gibson assembly mixtures were purchased from New England Biolabs. Thermo Scientific Phusion® DNA polymerase and T4 DNA ligase were used for all PCR reactions and ligations, respectively. PCR primers were supplied by Microsynth and are listed in the 'Oligonucleotides' column of Table S2. Commercial proteases were purchased from Applichem (proteinase K) and New England Biolabs (Endoproteinase GluC). Solvents for HPLC-MS analyses were Optima® LC-MS grade from Fisher Scientific and HPLC grade from Acros Organics and Sigma-Aldrich. Unless otherwise stated, chemicals were purchased from Sigma-Aldrich.

### Example 1 - Microvirgula aerodenitrifians transformation

*M. aerodenitrificans* DSMZ 15089 primary cultures containing 20 mL nutrient broth (NB) medium (5.0 g peptone, 3.0 g meat extract per 1.0 L) were inoculated from a glycerol stock and grown in a shaker to saturation for 1 day at 180 rpm and 30 °C. *E. coli* SM10 strains harboring various plasmids were grown overnight to saturation in 20 mL LB at 250 rpm and 37 °C. Both strains were harvested by centrifugation (10,000 x g), washed with a 0.9% (w/v) NaCl solution, and resuspended in 0.9% (w/v) NaCl solution that was then adjusted to an OD₆₀₀ of 4.0. Ratios of donor (SM10) and recipient (*M. aerodenitrificans*) strains of 1:9, 3:7, and 1:1 (v/v) were prepared and vortexed in a 1.0 mL final volume, spun down at 16,000 × g for 1 min, and resuspended in 50 µL 0.9% (w/v) NaCl solution. Cell mixtures were spotted on nutrient agar plates (1.5% (w/v) agar) and let dry prior to incubation at 37 °C for two days. The resulting mixed-cellular growths of different ratios were then removed from the plate with a sterile loop and transferred into 1.0 mL of a 0.9% (w/v) NaCl solution. Cell solutions (100 µL) were then plated out on selective NA plates containing gentamycin (10 µg/mL final concentration; positive selection for the pLMB509 plasmid) and carbenicillin (400 µg/mL final concentration; negative selection for SM10). Plates were incubated at 30 °C for up to 2 days.

### Example 2 - Culturing conditions

*M. aerodenitrificans:* Starter cultures (20 mL NB with 10 µg/mL gentamycin) were inoculated from a glycerol stock or a fresh colony harboring pLMB509-derived plasmids and grown overnight at 30 °C and 180 rpm. 200 µL of the culture was used to inoculate freshly prepared Terrific Broth (TB) media (20 mL with 10 µg/mL gentamycin) and grown overnight. 4 mL of the cultures was then used to inoculate 400 mL of TB media in 2 L Erlenmeyer flasks, grown at 30 °C and 180 rpm for 1-4 days. The cells were harvested via centrifugation, flash frozen in liquid nitrogen and stored at -80 °C until use.
*E. coli*: Plasmids were transformed in BL21 Star (DE3) unless otherwise stated and expression cultures were inoculated from overnight cultures in a 1:100 (v%v) dilution in 1 L TB medium. Cells were grown at 37 °C, 250 rpm to OD₆₀₀ 1.6-2 in 2.5 L Ultra Yield Flasks (Thompson). Flasks were then chilled in an ice bath for 30 min followed by addition of 1 mM IPTG (final concentration) and incubation at 16 °C, 250 rpm for 18 hours, unless otherwise stated.

### Example 3 - Protein purification

For all AerA variants, the same lysis method was used: Cells were resuspended in lysis buffer (20 mM imidazole, 50 mM sodium phosphate pH 8.0, 300 mM NaCl, 10% (v/v) glycerol) supplemented with 0.01% (v/v) Triton X-100 and 1 mg/mL lysozyme (Carl Roth) (final concentrations) in a ratio of 1 g wet cell weight to 4 mL lysis buffer. Cell suspensions were incubated at 37 °C and 250 rpm for 30 min and sonicated using a Qsonica Q700 sonicator with a 6 mm probe for 15 cycles of 10 s pulse/10 s rest at 25% amplitude followed by centrifugation at 18,000 × g (4 °C, 30 min). The resulting supernatant was incubited with 0.5-1 mL Protino Ni-NTA resin (Macherey-Nagel) for 1 h at 4 °C with gentle rocking.The Ni-NTA resin was then pelleted at 800 × g for 15 min, transferred to a fritted column, and washed with 1 round of 15 mL lysis buffer prior to protein elution with 2 rounds of 0.5-1.0 mL elution buffer (250 mM imidazole, 50 mM sodium phosphate pH 8.0, 300 mM NaCl, 10% (v/v) glycerol). When required, the elution fraction was concentrated sufficiently with Amicon Ultra centrifugal filters (3k or 5k MWCO, Millipore).

### Example 4 - Orthogonal D₂O-based induction system (ODIS) for labeling epimerized core peptides

Nhis-precursor peptides in pACYCDuet-1 was cotransformed with the AerD gene in pCDF-BAD/*Myc*-His A (pBAD/*Myc*-His A vector with the native origin of replication replaced by that of pCDFDuet) in *E. coli* BL21 (DE3) cells and plated on LB agar containing chloramphenicol (25 µg/mL) and ampicillin (100 µg/mL) and grown for 20 h at 37 °C or until colonies appeared. These colonies were used to inoculate 20 mL LB with chloramphenicol (25 µg/mL) and ampicillin (100 µg/mL) and grown overnight. The following day, nine separate 50 mL falcon tubes containing TB media (15 mL), chloramphenicol (25 µg/mL) and ampicillin (100 µg/mL) were inoculated with 150 µL and shaken at 37 °C, 250 rpm to OD₆₀₀ 1.6-2. Cultures were cooled on ice for 30 minutes, induced with IPTG (0.1 mM final concentration), and shaken (200 rpm, 16 °C) for 16 hours. The cultures were centrifuged (20 minutes, 10,000 x g) and the supernatant removed. The cell pellets were then washed with TB medium (2 x 15 mL) to remove any residual IPTG. In the second wash, the cells were shaken (200 rpm, 16 °C) for 1 hour to further metabolize intracellular IPTG. The washed cell pellets were resuspended in 15 mLTB medium in D₂O containing ampicillin (100 µg/mL in D₂O), and L-arabinose (100 L, 20% w/v in D₂O) and shaken (200 rpm, 16 °C) for 18 hours. The cultures were combined and centrifuged (30 minutes, 15,000 x g) and the pellet resuspended in 10 mL lysis buffer and treated as described in example 4. **Example 5** - Proteolytic cleavage for analysis of core peptides and generation of the core region

GluC cleavage: To analyse the post-translational modifications on the core peptide, between 20-40 µl of the elution fraction was mixed with 50 µl 2x GluC buffer and 10 µl GluC (0.25 µg/mL) to have a final volume of 100 µl and incubated at 37 °C for 16 hrs before analysis by LC-MS.

Proteinase K digest: 16 µl of the elution was mixed with 20 µl of proteinase K buffer (100 mM Tris, 4 mM CaCl₂, pH 8.0) 4 µl of proteinase K (2 mg/mL). For the elutions arising from expression in *E. coli,* this reaction was carried out in PCR tubes (12 h, 50 °C), while for elutions from expression in *M. aerodenitrificans* was carried out in glass inlets (12 h, 37 °C).

AerH digest: For small-scale reactions, typically 13 µl of the peptide elutions were mixed with 7 µl of Nhis-AerH (23 mg/ml) and 20 µl of proteinase K buffer. For large scale reactions, 2.4 mL of the peptide elution was mixed with 200 µl of Nhis-AerH and 2.6 mL of proteinase K buffer. All reactions were done in glass vials. The reaction was then spun down in glass tubes (2,000 x g, 20 min) with the supernatant collected and the pellet being redissolved in 2 mL propanol. This was again centrifuged (2,000 x g, 20 min) and the supernatant collected.

### Example 6 - Glucuronidase activity assay

Culture volumes equaling an OD₆₀₀ of 20 were centrifuged (10,000 x g, 10 mins) and the pellets resuspended in 1 mL lysis buffer (50 mM phosphate buffer pH 7.0, 5 mM dithiothreitol, 0.1% Triton X-100, 1 mg/ml lysozyme). Lysis was performed at 37 °C for 15 min followed by sonication using a Qsonica Q700 sonicator and 4420 microtip for 10 cycles of 10 s pulse/10 s rest at 25% amplitude. Lysates were centrifuged at 10,000 x g for 10 min. Then, 0.5 ml of lysate was supplemented with 10 µl 10 mg/mL X-glucuronide (5-Bromo-4-chloro-3-indolyl β-D-glucuronide) and incubated for 1 hour at 37 °C.

### Example 7 - Preparation of pyranine-encapsulated LUV's

To create large unilamellar vesicles (LUVs) a solution of 27.5 mg 1,2-Dimyristoyl-sn-glycero-3-phosphorylcholine (DMPC) and 8 mg cholesterol in CHCl₃ was dried to completeness under vacuum to form a thin lipid layer. The thin layer was suspended in 2 ml of trisodium 8-hydroxypyrene-1,3,6-trisulfonate (pyranine)-containing buffer (15 mM Hepes, pH 6.5, 200 mM NaCl, 1 mM pyridine) by mild sonication under argon gas. After five-times freeze-thaw cycles in liquid nitrogen, the lipid suspension was extruded 30 times through a polycarbonate filter with a pore size of 0.2 µm using the Avanti Mini Extruder (Avanti Polar Lipids, Alabaster, AL, USA). Residual external pyranine dye was subsequently removed by size exclusion chromatography using a PD-10 desalting column. The resulting solution was adjusted to 1 mM with dye free resuspension buffer (15mM Hepes pH 6.5, 200mM NaCl). For the H⁺/Na⁺ exchange assay the liposome solution was diluted to 50 µM with assay buffer (15 mM HEPES pH 7.5, 200 mM NaCl) to create a pH gradient.

### Example 8 - H⁺/Na⁺ Exchange Assay

A suspension of pyranine-loaded LUV's was placed into a quartz cuvette (2ml). The fluorescence emission was measured at 511 nm with an excitation at 460 nm in a Varian Cary Eclipse spectrofluorimeter. After 60s, peptides in DMSO were added at indicated concentrations and the fluorescence emission was recorded for 15 min at a sampling rate of 0.1 s. Afterwards LUVs were completely lysed by the addition of 5 µl of a 10% Triton X-100 aqueous solution. The background drift by the addition of pure DMSO was subtracted from all traces. The data was normalized against 100% lysis by Triton X-100.

### Example 9 - Cell-free assay

Wild-type *M. aerodenitrificans* was grown in 200 mLTB media at 30 °C for one day. 30 mL of the culture was centrifuged at 18,000 x g for 30 minutes and the cell pellet was resuspended in 1 mL ammonium acetate buffer (50mM ammonium acetate, 10% v/v glycerol and 50 mM potassium chloride, pH 5). The cells were then lysed using Qsonica Q700 sonicator and 4420 microtip for 10 cycles of 10 s pulse/10 s rest at 25% amplitude. Lysates were centrifuged at 11,000 x g for 30 min and the supernatant collected. To 1 mL of the lysate supernatant, 100 µL of Nhis-AerAD from *E. coli* was added and incubated for 2 days followed by affinity purification as described above. After purification, the sample was treated by gluC and analysed by LC-MS.

### Example 10 - Cytotoxic assays

The activity of aeronamide A was measured against HeLa cells. Stocked HeLa cells were resuspended in 10 mL HEPES buffered high glucose Dulbecco's Modified Eagle Medium (DMEM) supplemented with GlutaMAX (Gibco). Additionally, the medium contained 10% fetal calf serum (FCS) and 50 mg/mL gentamycin. The cells were centrifuged for 5 min at 1000 x g and room temperature. The medium was discarded and the cells resuspended in 10 mL fresh medium. The cells were put in a culture dish and incubated for 3-4 days at 37 °C. The cells were checked under the microscope and treated further only when 60-80% of the surface was covered with cells. The medium was removed from the culture flask and the cells were washed with 10 mL phosphate buffered saline (PBS). The PBS was discarded and the cells treated with 2 mL Trypsin-EDTA solution. When the cells were detached, 10 mL of medium was added and centrifuged for 5 min at 1000 x g and room temperature. The supernatant was discarded and 10 mL fresh medium were added. 2 mL of the cell suspension were put in a fresh culture flask containing 10 mL medium. Cells healthy enough for cytotoxicity assays were counted and diluted to a 10,000 cells/mL solution. 96 well plates were filled with 200 µL cell suspension per well. All plates were incubated overnight at 37 °C. The outer wells were not used for the assay. 2 µL of test solutions in DMSO were put in the B lane wells. Aeronamide A was a 1 mM solution, doxorubicin was used as a positive control at 1 mg/mL, and DMSO was used as negative control. 50 µL of lane B were transferred into lane C and mixed, and this transfer to the adjacent lane was repeated until lane G. The plates were then incubated for 3 days. 50 µL of 3-(4,5- dimethylthiazol-2-yl)-2,5-diphenyltetrazolium bromide (MTT) (1 mg/mL in water) were added to all wells and incubated for 3 h at 37 °C. The supernatant was discarded and 150 µL of dimethyl sulfoxide (DMSO) were added to all wells. Absorbance was measured at 570 nm and IC₅₀ was calculated using GraphPad Prism 6 (GraphPad).

### Example 11 - HPLC-MS analysis

For all HPLC-MS analysis a Phenomenex Kinetex 2.6 µm C18 100 Å (150 x 4.6 mm) was used on a Dionex Ultimate 3000 UHPLC system coupled to a Thermo Scientific Q Exactive mass spectrometer. Unless otherwise stated, the columns were heated to 50 °C. For expression products derived from *E. coli* and AerAP expressions in *M. aerodenitrificans*, the solvents used were water with 0.1% (v/v) formic acid (solvent A) and acetonitrile with 0.1% (v/v) formic acid (solvent B). A general LC method was used in this case; LC method 1: at a flow rate of 0.5 mL/min, solvent B was 5% from 0 to 2 min, 5% to 98% from 2 to 15 min, 98% from 15 to 20 min, 98% to 5% from 20 to 22 min, and 5% from 22 to 24.5 min. For all other expressions in *M. aerodenitrificans*, the solvents used were water with 0.5% (v/v) formic acid (or 0.1% TFA) as solvent A and n-propanol 0.5% (v/v) formic acid (or 0.1% TFA) as solvent B. Two different methods were used with LC method 2: at a flow rate of 0.75 mL/min, solvent B was 25% from 0 to 2 min, 25% to 65% from 2 to 20 min, 98% from 20.5 to 30 min, 98% to 25% from 30 to 32 min, and 25% from 32 to 32.5 min. LC method 3: at a flow rate of 0.75 mL/min, solvent B was 25% from 0 to 2 min, 25% to 65% from 2 to 30 min, 98% from 30.5 to 40 min, 98% to 25% from 40 to 42 min, and 25% from 42 to 42.5 min. The corresponding methods used for each sample or batches of runs are noted in their respective sections. Unless otherwise stated, ESI-MS was performed in positive ion mode, with a spray voltage of 3500 V, a capillary temperature of 268.75 °C, probe heater temperature ranging from 350 °C to 437.5 °C and an S-lens level range between 50 and 70. Full MS was done at a resolution of 35,000 (AGC target 2e5, maximum IT 100 ms, range 600-2000 *m*/*z*). Parallel reaction monitoring (PRM) or data-dependent MSMS was performed at a resolution of 17500 (AGC target between 1e5 and 1e6, maximum IT between 100 ms and 250 ms, isolation windows in the range of 1.1 to 2.2 *m*/*z*) using a stepped NCE of 18, 20 and 22 or an NCE of 18. Scan ranges, inclusion lists, charge exclusions, and dynamic exclusions were adjusted as needed.

### Example 12- Purification of aeronamide

Supernatants from the AerH digest were combined and diluted to 5% propanol and passed through a Phenomenex Strata® C18-E (55 µm, 70 Å) 5 g / 20mL column. The column was then washed with 4 column volumes of Milli Q water followed by 1 column volume of acetonitrile. Aeronamides were then eluted with 3 column volumes of n-propanol and evaporated using GeneVac EZ-2 Elite. The resulting pellet was dissolved in 75% propanol and separated by RP-HPLC (Phenomenex Luna 5µ C18, 10 x 250 mm, 2.4 mL/min, 200 nm) with a gradient elution from 25% n-propanol to 65% n-propanol from 2 to 30 min, with fractions collected and analyzed by LC-MS. Aeronamide A eluted between 26.5-27.5 min.

### Summary of the examples

pLMB509 was first developed as regulatable expression vector for use in Alphaproteobacteria (Appl Environ Microbiol 2012, 78(19): 7137-7140). The vector is derived from pRU1097 (pBBR origin of replication) and has an origin of transfer enabling conjugation and gentamycin resistance. For protein expression, a taurine inducible promoter system is present with a downstream *gfpmut3.1* reporter gene. To test for expression of the *aer* cluster in *M. aerodenitrificans*, the vector pLMB509 was modified by replacing the taurine induction system and *gfpmut3.1* with the *aer* promoter (362 bp upstream region from *aerC*) proceeded by the reporter gene *gusA,* encoding the enzyme glucuronidase A (example 6). This modified vector was transformed into M. *aerodenitrificans* and grown under different conditions. These conditions included LB- luria bertani medium, TB- terrific broth, NB- nutrient broth, MB- marine broth and temperatures of 30 °C and 37 °C, with samples being collected at day 1, day 2 and day 3 and frozen. The frozen samples were lysed, centrifuged and the supernatant was incubated with X-glucuronide (5-Bromo-4-chloro-3-indolyl β-D-giucuronide) for 1 hour. Of the conditions tested, cultivation of the *M*. *aerodenitrificans* reporter strain over a period of three days at 30 °C in terrific broth (TB) medium, routinely used for protein expression in *E. coli,* resulted in strong induction of GusA activity already after one day (Fig. 3a). The activity of the *aer* cluster was further established by incubating Nhis-AerAD from *E. coli* with cell free lysate from *M. aerodenitrificans* as described in example 9 resulting in the methylation of all 5 asparagine residues (Fig. 3b, c).

The modified pLMB509 vector, with Nhis-AerAX under the control of the *aer* promoter was successfully transformed into *Microvirgula aerodenitrificans* as described in example 1. Nhis-AerX includes Nhis-AerA, Nhis-AerAR1, Nhis-AerAR2, Nhis-AerAR3, Nhis-AerAP, Nhis-AerA(GG), Nhis-AerAR1(GG), Nhis-AerAR2(GG), Nhis-AerAR3(GG), Nhis-AerAP(GG) and Nhis-AerAV(GG). AR1-3 correspond to the core peptide sequences from the *rhp* cluster; AP to the core from the *poy* cluster; AV to the *vep* cluster.

Transformed colonies were picked and grown at induction conditions as described in example 2 followed by protein purification using affinity chromatography (example 3). The purified protein was treated with endoproteinase GluC (example 5) and analyzed by liquid chromatography-mass spectrometry/mass spectrometry (LC-MS/MS) to characterize the sites of modifications (Fig 4 and 5). In all cases apart from AerAR3 and AerAV, multiple sites carrying C- and N-methylations (catalyzed by *aerC* and *aerR* respectively) were localized. Thr1 dehydration (catalyzed by *aerF*) was observed for AerA, AerA(GG), AerAR1 and AerAR2. To localize epimerizations, ODIS (Orthogonal D₂O-based induction system, example 4) was performed. The precursor peptide sequences were expressed in *E. coli* followed by expression of the epimerase AerD in a deuterated background. Using this, an alternating pattern of epimerization was observed for all peptide sequences with AerA (21 epimerizations, Fig 2) and AerAR2 (23 epimerizations) undergoing full epimerization. For AerAR1 and AerAP 16 and 6 epimerizations were localized respectively.

To generate aeronamide A, Nhis-AerA purified from 5.5 L of *M.aerodenitrificans* was cleaved with Nhis-AerH purified from *E. coli* (example 5). The reaction mixture was purified using a C-18 solid phase exchange (SPE) column followed by high-pressure liquid chromatography (HPLC) to yield 600 µg of pure aeronamide A (Fig. 6a, example 12).

Aeronamide A showed potent cytotoxic activity against HeLa cells with an IC₅₀ value of 1.48 nM (polytheonamide B: 0.58 nM), but not towards the bacteria and fungi (example 10). To test whether the cytotoxicity is based on a similar pore-forming mechanism as for polytheonamides, an H⁺/Na⁺ ion exchange activity assay was performed on artificial liposomes (examples 7 and 8). Satisfyingly, a similar capability exhibited by polytheonamides for transporting H⁺ and Na⁺ ions was induced by aeronamide A (Fig. 6b). The almost picomolar range of activity was unexpected given that aeronamide A lacks the tert-butyl moiety on Thr1 (Fig. 6c), implicated as being a driving factor in polytheonamide cytotoxicity.

## Claims

**1.** An isolated and purified nucleic acid, comprising or consisting of a nucleic acid sequence selected from the group consisting of:
(i) a nucleic acid sequence listed in any one of SEQ ID NOs: 1 (aerC), 3 (aerD), 5 (aerF), 7 (aerE);
(ii) a nucleic acid sequence of at least 80 or 90 % sequence identity, optionally at least 95 % or 98 % sequence identity with a nucleic acid sequence of (i), optionally over the whole sequence;
(iii) a nucleic acid sequence that hybridizes to a nucleic acid sequence of (i) or (ii) under stringent conditions;
(iv) a fragment of any of the nucleic acid sequences of (i) to (iii), that hybridizes to a nucleic acid sequence of (i) or (ii) under stringent conditions;
(v) a nucleic acid sequence degenerated with respect to a nucleic acid sequence of any of (i) to (iv);
(vi) a nucleic acid sequence, wherein said nucleic acid sequence is derivable by substitution, addition and/or deletion of at least one nucleic acid of the nucleic acid sequences of (i) to (v) that hybridizes to a nucleic acid sequence of (i) or (ii) under stringent conditions;
(vii) a nucleic acid sequence complementary to the nucleic acid sequence of any of (i) to (vi);
wherein the nucleic acid sequence of any of (i) to (vii),
(a) when based on SEQ ID NO: 1 (aerC) encodes a polypeptide that has cobalamindependent rSAM methytransferase activity, optionally methylates one or more valine(s) to tert-leucine(s), methylates one or more isoleucine(s), methylates one or more leucine(s) and/or methylates one or more threonine(s);
(b) when based on SEQ ID NO: 3 (aerD) encodes a polypeptide that has rSAM epimerase activity to convert one or more L-amino acid(s) into D-amino acid(s);
(c) when based on SEQ ID NO: 5 (aerF) encodes a polypeptide that has dehydratase activity to dehydrate an N-terminal threonine or serine to an alpha-keto functional group; or
(d) when based on SEQ ID NO: 7 (aerE) and encodes a polypeptide that has asparagine (ASN) N-methyltransferase activity for methylating one or more side chain amines of one or more asparagine(s).

**2.** An isolated and purified **polypeptide** selected from the group consisting of:
(v) polypeptides comprising or consisting of an amino acid sequence selected from the group consisting of SEQ ID NOs: 2, 4, 6 and 8,
(vi) polypeptides encoded by a nucleic acid of claim 1;
(vii) polypeptides having an amino acid sequence identity of at least 70 or 80 %; optionally at least 90 or 95 % with the polypeptides of (i) and/or (ii); and
(viii) a functional fragment and/or functional derivative of (i), (ii) or (iii);
wherein the polypeptide of any of (i) to (iv),
(e) when based on an amino acid sequence of SEQ ID NO: 2 (AerC) has cobalamin-dependent rSAM methytransferase activity, optionally methylates one or more valine(s) to tert-leucine(s), methylates one or more isoleucine(s), methylates one or more leucine(s) and/or methylates one or more threonine(s);
(f) when based on an amino acid sequence of SEQ ID NO: 4 (AerD) has rSAM epimerase activity to convert one or more L-amino acid(s) into D-amino acid(s);
(g) when based on an amino acid sequence of SEQ ID NO: 6 (AerF) has dehydratase activity to dehydrate an N-terminal threonine or serine to an alpha-keto functional group; or
(h) when based on an amino acid sequence of SEQ ID NO: 8 (AerE) has asparagine (ASN) N-methyltransferase activity for methylating one or more side chain amine(s) of asparagine(s).

**3.** An antibody, optionally a monoclonal antibody, a functional fragment or functional derivative thereof, or antibody-like binding protein that specifically binds a polypeptide of claim 2.

**4.** A vector, optionally a plasmid, comprising a nucleic acid according to claim 1 and optionally a polypeptide for posttranslational modification by at least one protein according to claim 2.

**5.** A bacterial host cell, optionally a bacterial host cell producing cobalamin, optionally *microvirgula aerodenitricans* or *E. coli* host cell, optionally a cobalamin-producing *E. coli,* comprising a nucleic acid according to claim 1, wherein the host cell expresses and modifies a heterologous polypeptide by one or more polypeptides according to claim 2.

**6.** A *microvirgula aerodenitrificans* host cell, wherein the host cell expresses at least one heterologous polypeptide for enzymatic modification and one or more polypeptides according to claim 2, thereby modifying the at least one heterologous polypeptide by the one or more polypeptides according to claim 2.

**7.** A *microvirgula aerodenitrificans* host cell according to claim 6 expressing
(i) at least one polypeptide based on amino acid sequence SEQ ID NO: 2 (AerC) according to claim 2;
(ii) at least one polypeptide based on amino acid sequence SEQ ID NO: 4 (AerD) according to claim 2;
(iii) at least one polypeptide based on amino acid sequence SEQ ID NO: 6 (AerF) according to claim 2; and/or
(vi) at least one polypeptide based on amino acid sequence SEQ ID NO: 8 (AerE) according to claim 2,
with the proviso that expression of polypeptide (v) requires the expression of polypeptide (ii).

**8.** An *Escherichia coli* host cell according to claim 6 expressing
(i) at least one polypeptide based on amino acid sequence SEQ ID NO: 2 (AerC) according to claim 2;
(ii) at least one polypeptide based on amino acid sequence SEQ ID NO: 4 (AerD) according to claim 2;
(iii) at least one polypeptide based on amino acid sequence SEQ ID NO: 6 (AerF) according to claim 2; and/or
(vi) at least one polypeptide based on amino acid sequence SEQ ID NO: 8 (AerE) according to claim 2,
with the proviso that (a) expression of polypeptide (iv) requires the expression of polypeptide (ii) and (b) expression of (i) requires bacterial production or supplement of cobalamin. (preferably D and E)

**9.** A *microvirgula aerodenitrificans* or *Escherichia coli* host cell according to any of claims 6 to 8 expressing a heterologous polypeptide for enzymatic modification selected from the group of polypeptide precursors of boceprevir, telapevir, glecaprevir, atazanavir, vancomycin, colistin, teixobactin, bacitracin, gramicidin A-D, goserelin, leuprolide, nateglidine, octreotide, thiostreptons, bottromycins polymyxin, actinomycin, nisin, protegrin, dalbavancin, daptomycin, enfurvirtide, oritavancin, teicoplanin and guavanin 2.

**10.** A *microvirgula aerodenitrificans* or *Escherichia coli* host cell according to any of claims 6 to 9 expressing a heterologous polypeptide for enzymatic modification encoded by a nucleic acid sequence comprised in the aerA cluster of *Microvirgula aerodenitrificans* and encompassing the nucleic acid sequence of Seq. ID. NO.: 9 or a nucleic acid sequence hybridizing thereto under stringent conditions.

**11.** A composition comprising at least one nucleic acid according to claim 1, at least one polypeptide according to claim 2, at least one vector according to claim 3, or at least one bacterial host cell according to any of claims 4 to 10.

**12.** A method for producing and modifying a heterologous (poly)peptide in a *Microvirgula aerodenitricans* cell or an *E. coli* cell, optionally a cobalamin-producing *E. coli* cell, comprising the steps of
(i) providing a *Microvirgula aerodenitricans* or *E. coli* host cell according to any of claims 5 to 10, optionally a cobalamin-producing *E. coli* host cell, functionally expressing
a. at least one polypeptide enzyme according to claim 2 and
b. at least one heterologous (poly)peptide of interest; and
(ii) co-expressing the at least one polypeptide enzyme according to claim 2 and the at least one heterologous (poly)peptide of interest;
(iii) and optionally co-expressing one or more further enzymes for modifying the at least one heterologous (poly)peptide of interest;
(iv) and optionally at least partially purifying the so-modified heterologous (poly)peptide,
wherein the at least one polypeptide enzyme according to claim 2 is capable of catalyzing at least one modification in the heterolologous (poly)peptide.

**13.** The method of claim 12, comprising the steps of
(i) providing a *Microvirgula aerodenitricans* or a cobalamin-producing *E. coli* host cell, functionally expressing
a. at least one Cbl-dependent rSAM polypeptide enzyme as defined in claim 2 and
b. at least one heterologous (poly)peptide of interest; and
(ii) co-expressing the at least one Cbl-dependent rSAM enzyme and the at least one heterologous (poly)peptide;
(iii) and optionally co-expressing one or more further enzymes for modifying the heterologous (poly)peptide of interest;
(iv) and optionally at least partially purifying the so-modified heterologous (poly)peptide of interest;
wherein the at least one Cbl-dependent rSAM enzyme methylates one or more valine(s) to tert-leucine(s), methylates one or more isoleucine(s), methylates one or more leucine(s) and/or methylates one or more threonine(s) in the at least one heterolologous (poly)peptide of interest.

**14.** The method according to any of claims 12 and 13, wherein the one or more further enzymes for modifying the heterologous (poly)peptide(s) in step (iii) of claims 12 and 13 are selected from the polypeptides according to claim 2.

**15.** The method according to any of claims 12 to 14, wherein the one or more further enzymes for modifying the heterologous (poly)peptide(s) in step (iii) of claims 12 and 13 are selected from the group consisting of PoyB, PoyC (rSAM C-methyltransferases), OspD, AvpD, PlpD, PoyD (epimerases), PlpXY (β-amino acid incorporation, and PtsY (S-methyltransferase).

**16.** The method according to any of claims 12 to 15, wherein the at least one heterologous (poly)peptide is selected from the group consisting of polypeptide precursors of boceprevir, telapevir, glecaprevir, atazanavir, vancomycin, colistin, teixobactin, bacitracin, gramicidin A-D, goserelin, leuprolide, nateglidine, octreotide, thiostreptons, bottromycins polymyxin, actinomycin, nisin, protegrin, dalbavancin, daptomycin, enfurvirtide, oritavancin, teicoplanin, guavanin 2.

**16.** The method according to any of claims 12 to 16, wherein at least one, two or all of (i) the heterologous (poly)peptide of interest, the polypeptide enzyme(s) according to claim 2 and/or the one or more further enzymes for modifying the heterologous (poly)peptide(s) are present in the form of host-integrated DNA and/or in the form of a plasmid.

**17.** A polypeptide, optionally a cytotoxin, an antibiotic polypeptide or antiviral polypeptide comprising a posttranslational modification selected from the group consisting of
(i) a methylation of one or more valine(s) to tert-leucine(s), a methylation of one or more isoleucine(s), a methylation of one or more leucine(s), a methylation of one or more threonine(s);
(ii) a conversion of one or more L-amino acid(s) into D-amino acid(s);
(iii) a hydrolyzation of an N-terminal dehydro-threonine or -serine to an alpha-keto functional group; and
(iv) a methylation of one or more side chain amine(s) of asparagine(s),
wherein the polypeptide is obtained by a method according to any of claims 12 to 16.

**18.** Use of a nucleic acid according to claim 1, a polypeptide according to claim 2, an antibody of claim 3, a vector of claim 4, a host cell according to any of claims 5 to 10, a composition of claim 11 or a method according to any of claims 12 to 16 for modifying a heterologous (poly)peptide in a bacterial host cell or bacterially derived cell-free system.
